(19) Europäisches Patentamt
European Patent Office
Office européen des brevets

(11) EP 3 178 458 A1

(12) **EUROPEAN PATENT APPLICATION**

(43) Date of publication:
**14.06.2017 Bulletin 2017/24**

(51) Int Cl.:
**A61F 13/537** (2006.01)

(21) Application number: **15198435.8**

(22) Date of filing: **08.12.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **The Procter & Gamble Company Cincinnati, OH 45202 (US)**

(72) Inventors:
• **KREUZER, Carsten Heinrich**
  **65824 Schwalbach am Taunus (DE)**
• **PERI, Andrea**
  **65824 Schwalbach am Taunus (DE)**

(74) Representative: **Heide, Ute**
  **Procter & Gamble Service GmbH**
  **IP Department**
  **Frankfurter Strasse 145**
  **61476 Kronberg im Taunus (DE)**

(54) **ABSORBENT ARTICLES WITH DISTRIBUTION SYSTEM**

(57) The invention relates to an absorbent article having a longitudinal dimension and a lateral dimension and comprising a distribution system with a fibrous web and a layer of non-consolidated fibers deposited on at least a part of the fibrous web, wherein the longitudinal portions of the fibrous web are at least partially folded over onto the layer of non-consolidated fibers.

Fig. 1

**Description**

FIELD OF THE INVENTION

The present disclosure is generally directed to absorbent articles having a distribution system and, is more specifically directed to, absorbent articles having a distribution system positioned between an absorbent core and a topsheet, and comprising a layer of non-consolidated fibers and a fibrous web at least partially enwrapping the layer of non-consolidated fibers.

BACKGROUND OF THE INVENTION

Typically, absorbent articles may comprise a topsheet, a backsheet, and an absorbent core positioned intermediate the topsheet and the backsheet. The absorbent articles may comprise an acquisition layer or material and/or a distribution material or system. The distribution material/system is able to receive liquid bodily exudates (i.e., menses, urine, and/or runny BM) and distribute and/or transfer them to the absorbent core, or portions thereof, in order to render the absorbent core more efficient and to distribute the liquid bodily exudates more evenly over the absorbent core. Some absorbent articles use non-consolidated, modified cellulose fibers as distribution material.

However, these modified cellulose fibers exhibit relatively low integrity as they have not been consolidated into a coherent web. Additionally, these layers of non-consolidated fiber, such as modified cellulose fibers, upon receiving compressive loads and/or other strains associated with wearing the absorbent article, may be subject to tearing and breaking, thereby reducing their effectiveness. Integrity may be especially impacted when the layer of non-consolidated fibers is in a wet state during wear of the article, with fibers being partly collapsed.

Also, it is relatively difficult to lay down a layer of non-consolidated fibers in a homogeneous manner, such that the fibers are evenly distributed across the layer. This applies especially when the basis weight of the layer of non-consolidated fibers is relatively low, which also means that the number of fibers per area is relatively low. Another draw-back with inhomogeneous fiber layer down relates to opacity. It has been found that layers of non-consolidated fibers may exhibit inhomogeneous and/or low opacity. Especially in combination with thin absorbent cores, particularly those which have a very high percentage of superabsorbent polymer particles and low percentage of so-called "airfelt" (cellulose fibers), low opacity of the distribution material may be perceived negatively by a wearer or caretaker. When the absorbent core has reduced thickness, including areas where little absorbent material is deposited, the overall absorbent article may become more transparent, leading to see-through of the skin of the wearer. This is often perceived as low quality by wearers or caretakers, also indicating that the absorbent capacity of the article maybe inferior. Inhomogeneous opacity is also often associated with low quality of the product.

During manufacturing of the absorbent article, laying down loose fibers as a non-consolidated layer can pose certain problems on manufacturing hygiene. Lay down of the fibers typically is done using vacuum, which is applied underneath a conveyor belt, on which the fibers are provided. Alternatively, the fibers can be laid down on a drum. Lay down of the fibers typically is done using vacuum, which is applied underneath the surface on which the fibers are provided. However, the fibers may "escape" from the layer of non-consolidated fibers prior to having been properly positioned (and typically sealed by attaching the backsheet and topsheet to each other around their perimeter) in the final absorbent article. Such fibers may deposit in other areas of the manufacturing equipment, thereby contaminating the equipment.

Hence, there is a need for a distribution system which overcomes the drawbacks outlined above with respect to product and manufacturing process.

SUMMARY OF THE INVENTION

The invention relates to an absorbent article having a longitudinal dimension and a lateral dimension and comprising a liquid permeable topsheet, a liquid impermeable backsheet and an absorbent core positioned between the topsheet and the backsheet. The absorbent article further comprises a distribution system positioned between the absorbent core and the topsheet. The distribution system comprises i) a fibrous web having a central portion and first and second longitudinal portions, the central portion and first and second longitudinal portions being substantially parallel with the longitudinal dimension of the absorbent article, and further comprises ii) a layer of non-consolidated fibers deposited on at least a part of the central portion of the fibrous web, the layer having a first and a second surface. The first and second longitudinal portions of the fibrous web are folded over the layer of non-consolidated fibers along the longitudinal dimension of the absorbent article, such that the first and second longitudinal portions are facing towards the second surface of the layer of non-consolidated fibers and the central portion is facing towards the first surface of the layer of non-consolidated fibers. Thereby, the layer of non-consolidated fibers is partly or fully enwrapped by the fibrous web.

The absorbent core of the absorbent article may comprise at least 85% of superabsorbent polymers, by total weight of the absorbent material comprised by the absorbent core.

The absorbent core may comprise one or more area(s) substantially free of absorbent material.

BRIEF DESCRIPTION OF THE DRAWINGS

The above-mentioned and other features and advantages of the present disclosure, and the manner of attaining them, will become more apparent and the disclosure itself will be better understood by reference to the following description of non-limiting forms of the disclosure taken in conjunction with the accompanying drawings, wherein:

Fig. 1 is a top view of an absorbent article in the form of a diaper with some layers partially removed and comprising a distribution system in accordance with the present disclosure;

Fig. 2 is a cross-sectional view of the absorbent article, taken about line 2---2 of Fig. 1, in accordance with the present disclosure;

Fig. 3 is a top view of an absorbent article in the form of a diaper with some layers partially removed and comprising a distribution system in accordance with the present disclosure;

Fig. 4 is a cross-sectional view of the absorbent article, taken about line 4---4 of Fig. 3, in accordance with the present disclosure;

Fig. 5 is a cross-sectional view of the absorbent article, taken about line 4---4 of Fig. 3, where channels have formed as a result the absorbent article being loaded with liquid bodily exudates in accordance with the present disclosure;

Fig. 6 is an example plan view of a wet-laid, three-dimensional fibrous web of a distribution system in accordance with the present disclosure;

Fig. 7 is a cross-sectional view of the three-dimensional fibrous web taken about line 7-7 of Fig. 6, in accordance with the present disclosure;

Fig. 8 is another example plan view of a wet-laid, three-dimensional fibrous web of the distribution material in accordance with the present disclosure;

Fig. 9 is an cross-sectional view of the three-dimensional fibrous web taken about line 9-9 of Fig. 8, in accordance with the present disclosure;

Fig. 10 is an example plan view of a portion of a papermaking belt used to make a fibrous web of a distribution system in accordance with the present disclosure;

Fig. 11 is an example of a patterned film used in the process of creating a papermaking belt in accordance with the present disclosure;

Fig. 12 is an example of a raised resin portion of a papermaking belt in accordance with the present disclosure;

Fig. 13 is an example of a fibrous web production process in accordance with the present disclosure;

Fig. 14 is a top view of an absorbent article that is a sanitary napkin with some of the layers cut away in accordance with the present disclosure;

Fig. 15 is a side view of a package of absorbent articles showing the package width. The outer surface is illustrated as transparent for purposes of clarity.

DETAILED DESCRIPTION

Various non-limiting forms of the present disclosure will now be described to provide an overall understanding of the principles of the structure, function, manufacture, and use of the absorbent articles having distribution materials disclosed herein. One or more examples of these non-limiting forms are illustrated in the accompanying drawings. Those of ordinary skill in the art will understand that the absorbent articles having distribution materials described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

**Definitions**

As used herein, "absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.

As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

As used herein, "diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant maybe preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

As used herein, a "pantiliner" and a "sanitary napkin" generally have two end regions and a middle region (i.e. a crotch region). The pantiliner and the sanitary napkin has a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins. Sanitary napkins and pantiliners of the present invention comprise barrier leg cuffs.

As used herein, a "nonwoven web" is a manufactured web of directionally or randomly oriented fibers, consolidated and bonded together. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. Nonwoven webs may be bonded by heat and/or pressure or may be adhesively bonded. Bonding may be limited to certain areas of the nonwoven web (point bonding, pattern bonding). Nonwoven webs may also be hydro-entangled or needle-punched. The basis weight of nonwoven fabrics is usually expressed in grams per square meter ($g/m^2$).

As used herein, the term "wet-laid" is a process step in papermaking. In the wet-laid process, cellulose fibers are first mixed with chemicals and water to obtain a uniform dispersion called a slurry at very high dilutions of 0.01 percent weight to 0.5 percent weight of the fibers. The slurry is then deposited on a moving foraminous member (or wire screen) where the excess water is drained off, leaving the fibers randomly laid in a uniform web, which is then bonded and finished as required.

As used herein, the term "wet-formed" refers to wet-laid fibrous webs that have a three-dimensional surface topography imparted to them by the papermaking process of the present disclosure.

As used herein, the term "cellulosic fiber" refers to natural fibers which typically are wood pulp fibers. Applicable wood pulps comprise chemical pulps, such as Kraft, sulfite, and sulfate pulp, as well as mechanical pulps comprising, for example, groundwood, thermomechanical pulp and chemically modified thermomechanical pulp. Pulps derived from both deciduous trees (hereinafter, also referred to as "hardwood") and coniferous trees (hereinafter, also referred to as "softwood") may be utilized. The hardwood and softwood fibers may be blended, or alternatively, may be deposited in layers to provide a stratified web.

As used herein, the term "fibrous web" refers to an individual, self-sustaining, integral web. The web may comprise cellulose fibers. The web maybe wet-laid.

As used herein, the term "caliper" refers to the thickness of a web under a defined load, e.g. at 2.06 kPa.

As used herein, "laterally outboard" and "laterally outward" as used herein in relation to the distribution system describes the position of a component or material relative to another component or material with respect to the lateral axis of the absorbent article in which the distribution system has been provided. Laterally outboard and laterally outward means further away from the lateral axis. If the fibrous web of the distribution system extends laterally outboard or laterally outward of the layer of non-consolidated fibers, one or both of the two end edges of the fibrous web is further away from the lateral axis of the absorbent article in which the distribution system has been provided compared to the respective end edges of the layer of non-consolidated fibers.

As used herein, the term "machine direction" (or MD) is the direction parallel to the flow of a material through a manufacturing line.

As used herein, the term "cross-machine direction" (or CD) is the direction perpendicular to the machine direction.

As used herein, the term "three-dimensional" means that the fibrous web has a three-dimensional surface topography visual discernible to the naked eye of an observer. That is, an observer will be able to tell by observing the fibrous web with the naked eye that the fibrous sheet does not have a flat surface topography (having a two-dimensional surface topography within the meaning of the present invention), but the fibrous web adopts a three-dimensional surface topography when placed on a flat surface, such as the surface of a table having a smooth, even surface.

As used herein, the term "substantially continuous" regions refers to an area within which one can connect any two points by an uninterrupted line running entirely within that area throughout the line's length. That is, the substantially continuous region has a substantial "continuity" in all directions parallel to the first plane and is terminated only at edges of that region. The term "substantially," in conjunction with continuous, is intended to indicate that while an absolute continuity is preferred, minor deviations from the absolute continuity may be tolerable as long as those deviations do not appreciably affect the performance of the fibrous webs (or a papermaking belt) as designed and intended.

As used herein, the term "substantially semi-continuous" regions refer to an area which has "continuity" in all, but at least one, directions parallel to the first plane, and in which area one cannot connect any two points by an uninterrupted line running entirely within that area throughout the line's length. The semi-continuous framework may have continuity only in one direction parallel to the first plane. By analogy with the continuous region, described above, while an absolute continuity in all, but at least one, directions is preferred, minor deviations from such a continuity may be tolerable as long as those deviations do not appreciably affect the performance of the fibrous web.

As used herein, the term "discrete regions" refer to regions that are discontinuous and separated from other areas in all directions parallel to the first plane.

As used herein, the term "papermaking belt" refers to a structural element that is used as a support for the fiber or filaments that may be deposited thereon during a process of making a fibrous web, and as a forming unit to form a desired microscopical geometry of a fibrous web. The papermaking belt may comprise any element that has the ability to impart a three-dimensional pattern to the fibrous web being produced thereon, and includes, without limitation, a stationary plate, a belt, a cylinder/roll, a woven fabric, and a band.

As used herein, the terms "substantially free of absorbent material" and "substantially absorbent material free" mean that the basis weight of the absorbent material in the substantially absorbent material free areas is at least less than 10%, in particular less than 5%, or less than 2%, of the basis weight of the absorbent material in the rest of the absorbent core.

As used herein, the term "non-consolidated fibers" refers to fibers which are not formed into a self-sustaining, integral web.

**General Description of an Absorbent Article**

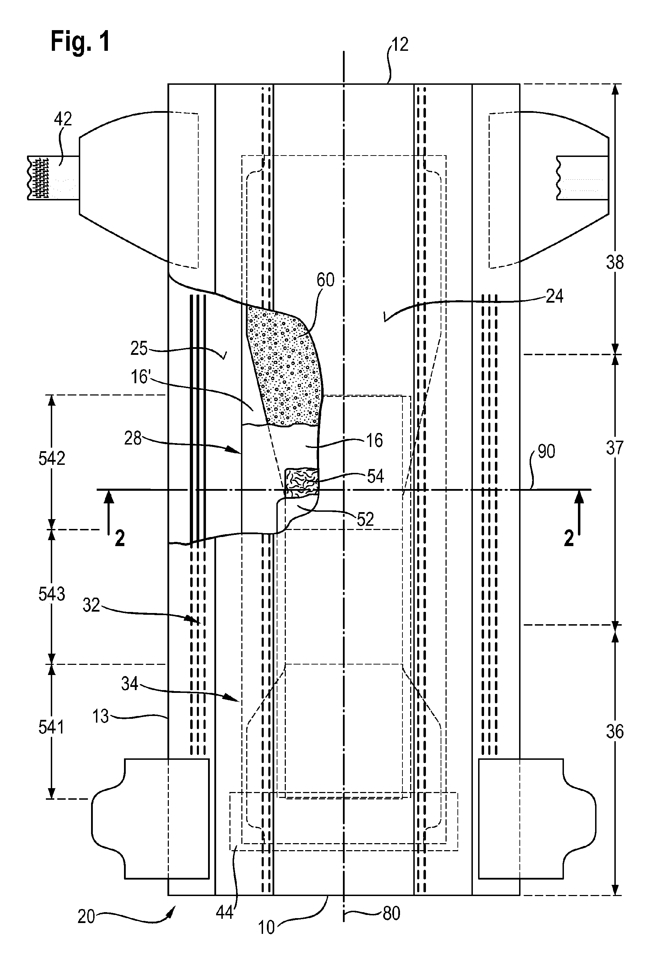

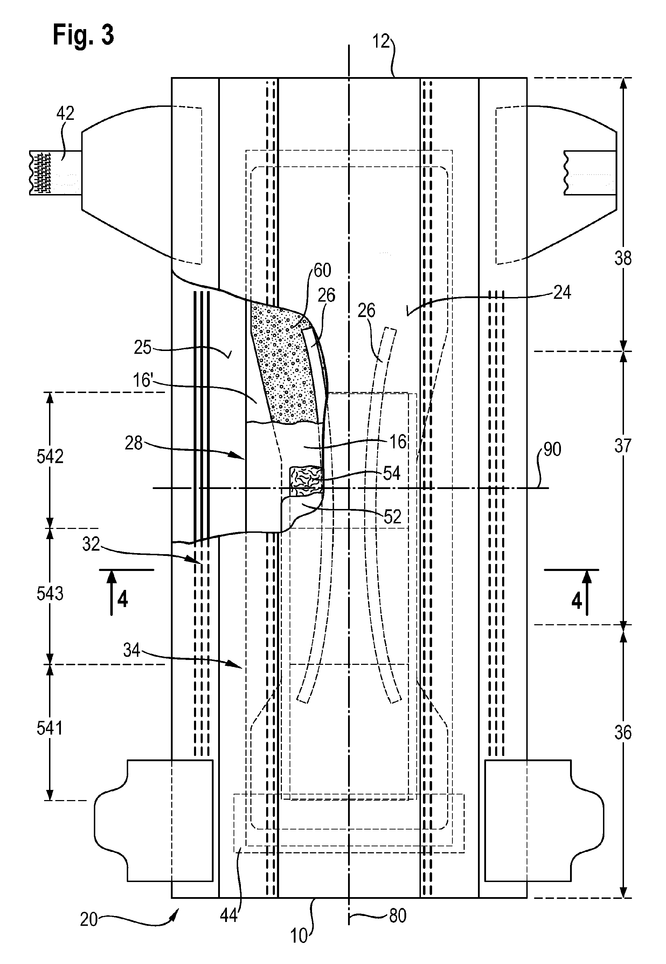

Referring to Figs. 1 and 3, example absorbent articles 20 are disclosed. Figs. 1 and 3 are top plan views of the absorbent articles 20 (in Fig. 1 and 3: a diaper), in a flat-out state, with portions of the structure being cut-away to more clearly show the construction of the absorbent articles 20. These absorbent articles 20 are shown for illustrative purposes only as the present disclosure may be used for making a wide variety of diapers or other absorbent articles. The absorbent article of Fig. 1 has a different core structure as the absorbent article of Fig. 3, as will be explained further below.

The absorbent article 20 comprises a liquid permeable topsheet 24, a liquid impermeable backsheet 25, an absorbent core 28 positioned intermediate the topsheet 24 and the backsheet 25, and a distribution system 54 comprising a fibrous web 120 and a layer of non-consolidated fibers 121. The absorbent article 20 comprises a front edge or waist edge 10, a back edge or waist edge 12, and two longitudinal side edges 13. The front edge 10 is the edge of the absorbent article 20 which is intended to be placed towards the front of the user when worn, and the back edge 12 is the opposite edge. The absorbent article 20 has a longitudinal dimension and a lateral dimension and may be notionally divided by a longitudinal axis 80 extending from the front edge 10 to the back edge 12 of the absorbent article 20 and dividing the absorbent article 20 in two substantially symmetrical halves relative to the longitudinal axis, when viewing the absorbent article 20 from the wearer-facing side in a flat, laid out configuration, as e.g. illustrated in Figs. 1 and 3.

The absorbent article 20 may be divided by a lateral axis 90 into a front half and a back half of equal length measured along the longitudinal axis 80, when the absorbent article 20 is in a flat, laid-out state. The absorbent article's lateral axis 90 is perpendicular to the longitudinal axis 80 and is placed at half the longitudinal length of the absorbent article 20.

The longitudinal dimension of the absorbent article extends substantially parallel to the longitudinal axis 80 and the lateral dimension extends substantially parallel to the lateral axis 90.

The absorbent article 20 may be notionally divided into a front region 36, a back region 38 and a crotch region 37 located between the front region 36 and the back region 38 of the absorbent article 20. Each of the front, back and crotch regions are 1/3 of the longitudinal dimension of the absorbent article 20.

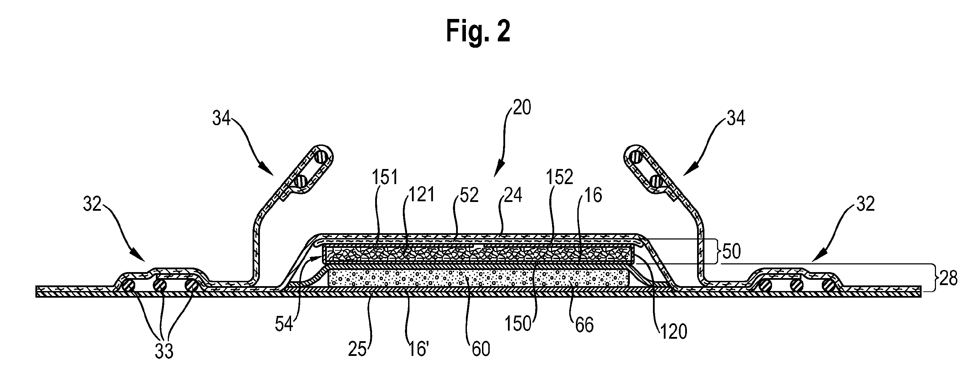

The absorbent article 20 may comprises a distribution system 54 comprising a fibrous web 120 and a layer of non-consolidated fibers 121, and the absorbent article may further comprise an acquisition layer or material 52 which may be placed on top of the distribution system 54 towards the topsheet (the acquisition material and distribution system are collectively referred to as acquisition-distribution system "ADS", designated as 50 in Fig. 2). Alternatively, the absorbent articles may only comprise a distribution system and no acquisition layer. The absorbent article 20 may comprise elasticized gasketing cuffs 32 and upstanding barrier leg cuffs 34. Figs. 1-4 also show other typical diaper components such as a fastening system comprising fastening tabs 42 positioned proximate to the back edge 12 of the absorbent article 20 and cooperating with a landing zone 44 positioned proximate to the front edge 10 of the absorbent article 20. The absorbent article 20 may also comprise other typical components, which are not represented in the Figures, such as a back elastic waist feature, a front elastic waist feature, transverse barrier cuff(s), and/or a lotion application, for example.

The topsheet 24, the backsheet 25, the absorbent core 28 and the other absorbent article components may be assembled in a variety of well known configurations, in particular by adhesive bonding and/or heat and/or pressure embossing. Example diaper assemblies are for example generally described in U.S. Pat. No. 3,860,003, U.S. Pat. No. 5,221,274, U.S. Pat. No. 5,554,145, U.S. Pat. No. 5,569,234, U.S. Pat. No. 5,580,411, and U.S. Pat. No. 6,004,306.

The absorbent core 28 may comprise an absorbent material 60 that is a blend of cellulosic fibers (so called "airfelt") and superabsorbent polymers in particulate form encapsulated in one or more webs, see for example U.S. Pat. No. 5,151,092 to Buell. Alternatively, the absorbent core 28 may be free of airfelt, or substantially free of airfelt, as described in further detail herein.

**Absorbent Core**

The absorbent core 28 may comprise an absorbent material 60 with a high amount of superabsorbent polymers (SAP) enclosed within a core wrap. The absorbent material 60 may comprise from 80% to 100%, 85% to 100%, or 90% to 100%, of SAP, such as SAP particles, by weight of the absorbent material 60. The core wrap is not considered as an absorbent material 60 for the purpose of assessing the percentage of SAP in the absorbent core 28.

The term "absorbent material" refers to a material which has at least some absorbency and/or liquid retaining properties, such as SAP, cellulosic fibers as well as some hydrophilically treated synthetic fibers. Typically, adhesives used in making absorbent cores have no absorbency properties and are not considered as absorbent material. The high SAP content of the absorbent material, as discussed above, may provide a relatively thin absorbent core 28 compared to conventional absorbent cores typically comprising between 40-60% SAP and 40-60% of cellulosic fibers. The absorbent material 60 may in particular comprise less than 15% weight percent, less than 10% weight percent, less than 5% weight percent, less than 3% weight percent, less than 1% weight percent, or may even be substantially free of natural and/or synthetic fibers. Figs. 1 and 2 are illustrations of an absorbent article 20 comprising an "airfelt-free" absorbent core 28.

"Airfelt-free" absorbent cores 28 comprising relatively high amount of SAP with various absorbent core designs have been proposed in U.S. Pat. No. 5,599,335 (Goldman), EP1447066A1 (Busam), WO 95/11652 (Tanzer), U.S. Pat. No. 5,650,214 (Handoff), and WO 2012/052172 (Van Moldered).

The absorbent core 28 may comprise one or more adhesives to help immobilize the SAP within the core wrap and/or to ensure integrity of the core wrap, in particular when the core wrap is made of one or more webs. In one form, the core wrap may extend over a larger area than strictly needed for containing the absorbent material 60 within.

**Core Wrap**

Again referring to Figs. 1-4, the absorbent material 60 may be at least partially encapsulated in one or more webs.

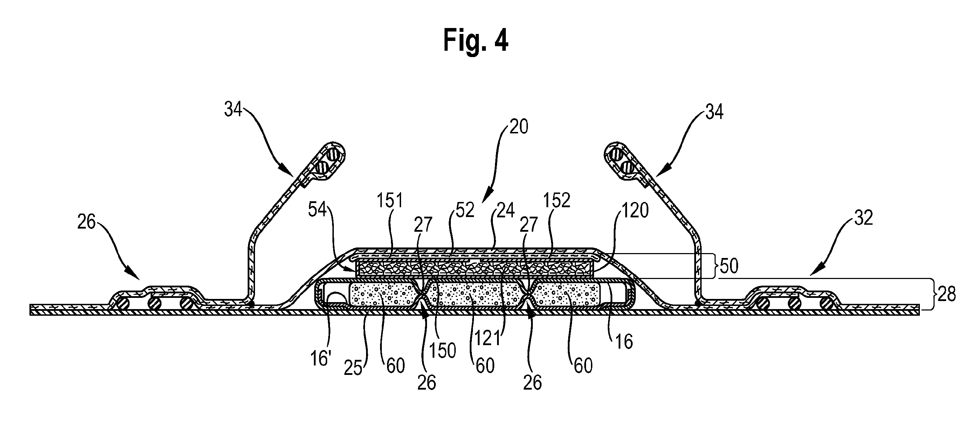

The core wrap may comprise a top side 16 facing the topsheet 24 and a bottom side 16' facing the backsheet 25. The core wrap may be made of a single web folded around the absorbent material 60. Alternatively, the core wrap may be made of two webs (one mainly providing the top side 16 and the other mainly providing the bottom side 16') which are attached to another, as shown in the example of Fig. 2. Typical configurations of the core wrap are the so-called C-wrap and/or sandwich wrap. Referring to Fig. 4, in a C-wrap, the longitudinal and/or lateral edges of one of the webs may be folded over another web to form flaps. These flaps may then be bonded to the external surface of the other web, typically by bonding with one or more adhesives.

The core wrap may be formed by any materials suitable for receiving and containing the absorbent material 60. Typical web materials used in the production of conventional absorbent cores may be used, in particular fibrous webs made of wet-laid fibers, films, wovens or nonwovens, or laminate of any of these. The core wrap may in particular be formed by a nonwoven web, such as a carded nonwoven, spunbond nonwoven ("S") or meltblown nonwoven ("M"), and laminates of any of these. For example, spunmelt polypropylene nonwovens are suitable, in particular those having a laminate web SMS, or SMMS, or SSMMS, structure, and having a basis weight range of about 5 g/m$^2$ to 20 g/m$^2$. Suitable materials are, for example, disclosed in U.S. Pat. No. 7,744,576, U.S. Pat. Publ. No. 2011/0268932A1, U.S. Pat. Publ. No. 2011/0319848A1, or U.S. Pat. Publ. No. 2011/0250413A1. Nonwoven materials provided from synthetic fibers may also be used, such as PE, PET and in particular PP.

The top side 16 of the core wrap maybe sealed to the bottom side 16' of the core wrap at least partially along all the edges of the absorbent core 28. The term "seal" is to be understood in a broad sense. The seal does not need to be continuous along the whole periphery of the core wrap, but may be discontinuous along part or the whole of it, such as formed by a series of closely spaced apart seal points on a line. Typically, a seal may be formed by adhesive bonding and/or thermal bonding.

**"Airfelt-free" Absorbent Core Having One or More Substantially Material Free Areas**

Referring generally to Figs. 3 and 4, the absorbent core 28 may comprise an absorbent material deposition area defined by the periphery of the layer formed by the absorbent material 60 within the core wrap.

The absorbent core 28 may comprise one or more substantially absorbent material free area(s) 26 or absorbent material free areas 26 (hereinafter together referred to as "substantially absorbent material free area(s)") which is/are substantially free of, or free of, absorbent material 60 and through which a portion of the top side 16 of the core wrap is attached by one or more core wrap bond(s) 27 to a portion of the bottom side 16' of the core wrap. In particular, there may be no absorbent material 60 in these areas. Minimal amounts such as contaminations with absorbent material 60 that may occur during the making process are not considered as absorbent material 60. The one or more substantially absorbent material free area(s) 26 is/are advantageously confined by the absorbent material 60, which means that the substantially absorbent material free area(s) 26 do(es) not extend to any of the edge of the absorbent material deposition area.

The portions of the top side 16 and the bottom side 16' of the core wrap maybe attached together continuously along the substantially absorbent material free area(s) 26. However, one or more core wrap bonds 27 along the substantially absorbent material free area(s) 26 may also be discontinuous (intermittent) such as series of point bonds. The core wrap bond(s) (27) may be provided by known attachment methods, such as adhesive bonding, pressure bonding, ultrasonic bonding, heat bonding, dynamic mechanical bonding, or combinations thereof.

In one form, the absorbent core 28 may comprise at least two substantially absorbent material free areas 26 symmetrically disposed on both sides of the longitudinal axis 80 or the lateral axis 90.

The substantially absorbent material free area(s) 26 may be straight and completely oriented longitudinally and parallel to the longitudinal axis 80, but also may be curved or have one or more curved portions. The substantially absorbent free area(s) 26 may also be oriented parallel to the lateral axis 90 or may be oriented in any other suitable direction.

Furthermore, in order to reduce the risk of liquid bodily exudate leakages, the substantially absorbent material free area(s) 26 advantageously do not extend to any of the edges of the absorbent material deposition area, and, therefore, may be surrounded by and fully encompassed within the absorbent material deposition area of the absorbent core 28. As an example, the smallest distance between a substantially absorbent material free area 26 and the closest edge of the absorbent material deposition area may be at least about 2 mm or at least about 5 mm, although other distances may also be suitable.

"Airfelt-free" absorbent cores 28 comprising substantially absorbent material free areas 26 have been proposed, see for example European Patent Application EP 2740449A1.

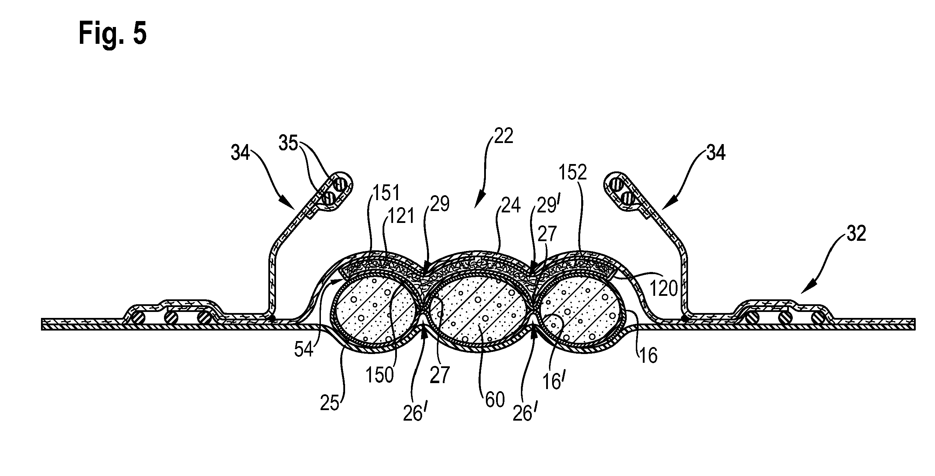

Referring to Fig. 5, one or more channel(s) 26' along the substantially absorbent material free area(s) 26 in the absorbent core 28 may start forming when the absorbent material 60 absorbs one or more liquid bodily exudates and begins to swell. As the absorbent core 28 absorbs more liquid, the depressions within the absorbent core 28 formed by the channel(s) 26' may become deeper and more apparent to the eye and the touch. The formation of the channel(s) 26' may also serve to indicate that the absorbent article 20 has been loaded with liquid bodily exudates. The core wrap bond(s) 27 should remain substantially intact at least during a first phase as the absorbent material 60 absorbs a moderate quantity of liquid bodily exudates (e.g., urine, menses, runny BM).

As shown in Fig. 5, when the absorbent material 60 swells, the core wrap bonds 27 remain at least initially attached in the substantially absorbent material free areas 26. The absorbent material 60 swells in the rest of the absorbent core 28 when it absorbs liquid bodily exudates, so that the core wrap thus forms channels 26' along the substantially absorbent material free areas 26 comprising the core wrap bonds 27.

When the absorbent material 60 in the absorbent core 28 swells, so that the channels 26' form, the surface of the top side 16 of the core wrap may become uneven, see Fig. 5. As the distribution material is placed on top of the top side 16 of the core wrap, the distribution material may follow the uneven surface of the top side 16. The formation of the channels 26' may create indentations where portions of the distribution material may sink into these indentations. This may promote the formation of disruptions of the distribution material, when the distribution material 54 is an air-laid material and in a wet state during use. Hence, "airfelt-free" absorbent cores 28 comprising substantially absorbent material free areas 26 may promote more disruptions in a distribution material made of air-laid, non-consolidated fibers when the absorbent material 60 swells.

To solve such a problem, a distribution system 54 is provided which comprises a fibrous web 120, such as a wet-laid or wet-formed fibrous web, which partly or fully enwraps a layer of non-consolidated fibers 121, such as cellulose fibers or modified cellulose fibers. A fibrous web, such as a wet-laid or wet-formed fibrous web, can provide the required wet strength to the distribution system and sustain the integrity of the layer of non-consolidated fibers during use of the absorbent article. The fibrous web may have a wet burst strength from about 50 g to about 500 g or from about 250 g to about 350 g or from about 300 g to about 350 g according to the Wet Burst Test Method as disclosed herein, may be provided to achieve a more wet integrated distribution system.

### Distribution System

The absorbent article 20 comprises a distribution system 54 positioned between the topsheet 24 and the absorbent core 28. An acquisition system may be provided in addition, which may be positioned between the topsheet and the distribution system, or, alternatively, may be provided between the distribution system 54 and the absorbent core 28.

### Wet-laid fibrous web

The fibrous web comprised by the distribution system may be wet-laid or wet-formed. It may comprise at least 80% cellulose fibers by weight of the fibrous web.

The wet-laid fibrous web may be produced by forming a predominantly aqueous slurry comprising about 90% to about 99.9% water or other suitable fluid or liquid. The non-aqueous component of the slurry used to make the wet-laid and/or wet-formed fibers may comprise from about 1% to about 95% or about 5% to about 80% of cellulosic fibers, such as eucalyptus fibers, by weight of the non-aqueous components of the slurry. The non-aqueous components may comprise from about 8% to about 60% of cellulosic fibers, such as eucalyptus fibers, by weight of the non-aqueous components of the slurry, or from about 15% to about 30% of cellulosic fibers, such as eucalyptus fibers, by weight of the non-aqueous component of the slurry. In some instances, the slurry may comprise about 45% to about 60% of Northern Softwood Kraft fibers with up to 20% Southern Softwood Kraft co-refined together, about 25% to about 35% unrefined Eucalyptus fibers and from about 5% to about 30% of either repulped product broke or thermo-mechanical pulp. Any other suitable cellulosic fibers and/or combinations thereof within the knowledge of those of skill in the art may also be used.

The wet-laid fibrous web may comprise a mixture of at least two different materials. At least one of the materials may comprise a non-naturally occurring fiber, such as a polypropylene fiber or a polyolefin fiber, for example, and at least one other material, different from the first material, comprising a solid additive, such as another fiber and/or a particulate, for example.

Synthetic fibers useful herein may comprise any suitable material, such as, but not limited to polymers, those selected from the group consisting of polyesters, polypropylenes, polyethylenes, polyethers, polyamides, polyhydroxyalkanoates, polysaccharides, and combinations thereof. More specifically, the material of the polymer segment may be selected from the group consisting of poly(ethylene terephthalate), poly(butylene terephthalate), poly(1,4-cyclohexylenedimethylene terephthalate), isophthalic acid copolymers (e.g., terephthalate cyclohexylene-dimethylene isophthalate copolymer), ethylene glycol copolymers (e.g., ethylene terephthalate cyclohexylene-dimethylene copolymer), polycaprolactone, poly(hydroxyl ether ester), poly(hydroxyl ether amide), polyesteramide, poly(lactic acid), polyhydroxybutyrate, and combinations thereof. Further, the synthetic fibers may be a single component fibers (i.e., single synthetic material or a mixture to make up the entire fiber), multi-component fibers, such as bicomponent fibers (i.e., the fiber is divided into regions, the regions including two or more different synthetic materials or mixtures thereof), and combinations thereof. Nonlimiting examples of suitable bicomponent fibers are fibers made of copolymers of polyester (polyethylene terephthalate/isophtalate/polyester (polyethylene terephthalate) otherwise known as "CoPET/PET" fibers, which are commercially available from Fiber Innovation Technology, Inc., Johnson City, TN.

Non-wood Pulp Fibers

The fibrous web may also comprise non-wood fibers.

Non-wood fibers may comprise fibers made from polymers, specifically hydroxyl polymers. Non-limiting examples of suitable hydroxyl polymers include polyvinyl alcohol, starch, starch derivatives, chitosan, chitosan derivatives, cellulose derivatives, gums, arabinans, galactans, and combinations thereof. Additionally, other synthetic fibers such as rayon, polyethylene, and polypropylene fibers can be used within the scope of the present disclosure. Other suitable materials are also intended to be within the scope of the present disclosure.

Non-wood fibers may also comprise fibers that comprise processed residuals from agricultural crops such as wheat straw, wetland non-tree plants such as bulrush, aquatic plants such as water hyacinth, microalgae such as Spirulina and macroalgae seaweeds such as red or brown algae. Examples of non-wood natural materials include, but are not limited to, wheat straw, rice straw, flax, bamboo, cotton, jute, hemp, sisal, bagasse, hesperaloe, switchgrass, miscanthus, marine or fresh water algae/seaweeds, and combinations thereof.

Optional Ingredients

To enhance permanent wet strength of the fibrous web of the distribution system 54, cationic wet strength resins may be added to the papermaking furnish or to the embryonic web. The fibrous web made of wet-laid fibers may comprise one or more cationic wet strength resins selected from the group consisting of a base activated epoxide polyamide epichlorohydrin resin, an urea-formaldehyde resin, a melamine formaldehyde resin, a polyamide-epichlorohydrin resin, a polyethyleneimine resin, a polyacrylamide resin, a dialdehyde starch and mixtures thereof.

From about 0.90 kg/ton to about 2.27 kg/ton, from about 0.22 kg/ton to about 13.6 kg/ton, or from about 4.53 kg/ton to about 11.34 kg/ton of dry paper fibers of the cationic wet strength resin may be used.

The cationic wet strength resins may comprise cationic water soluble resins. These resins may improve wet strength in a fibrous web. This resin may improve either temporary or permanent wet strength to the fibrous web. KYMENE® resins obtainable from Hercules Inc., Wilmington, Del. may be used, including KYMENE® 736 which is a polyethyleneimine (PEI) wet strength polymer. It is believed that the PEI may improve wet strength by ionic bonding with the pulps carboxyl sites. KYMENE® 557LX is polyamide epichlorohydrin (PAE) wet strength polymer. It is believed that the PAE contains cationic sites that may lead to resin retention by forming an ionic bond with the carboxyl sites on the pulp. The polymer contains 3-azetidinium groups which react to form covalent bonds with the pulps' carboxyl sites as well as with the polymer backbone. The product may undergo curing in the form of heat or undergo natural aging for the reaction of the azentidinium group. KYMENE® 450 is a base activated epoxide polyamide epichlorohydrin polymer. It is theorized that like 557LX the resin attaches itself ionically to the pulps' carboxyl sites. The epoxide group is much more reactive than the azentidinium group. The epoxide group reacts with both the hydroxyl and carboxyl sites on the pulp, thereby giving higher wet strengths. The epoxide group may also crosslink to the polymer backbone. KYMENE® 2064 is also a base activated epoxide polyamide epichlorohydrin polymer. It is theorized that KYMENE® 2064 may improve its wet strength by the same mechanism as KYMENE® 450. KYMENE® 2064 differs in that the polymer backbond contains more epoxide functional groups than does KYMENE® 450. Both KYMENE® 450 and KYMENE® 2064 may require curing in the form of heat or natural aging to fully react all the epoxide groups, however, due to the reactiveness of the epoxide group, the majority of the groups (80-90%) react and improve wet strength off the paper machine. Mixtures of the foregoing may be used. Other suitable types of such resins include urea-formaldehyde resins, melamine formaldehyde resins, polyamide-epichlorohydrin resins, polyethyleneimine resins, polyacrylamide resins, dialdehyde starches, and mixtures thereof. Other suitable types of such resins are described in U.S. Pat. No. 3,700,623, issued Oct. 24, 1972; U.S. Pat. No. 3,772,076, issued Nov. 13, 1973; U.S. Pat. No. 4,557,801, issued Dec. 10, 1985 and U.S. Pat. No. 4,391,878, issued July 5, 1983.

The cationic wet strength resin may be added at any point in the process, where the resin will come in contact with the fibers prior to forming the wet web.

Fibrous web having three-dimensional surface topography

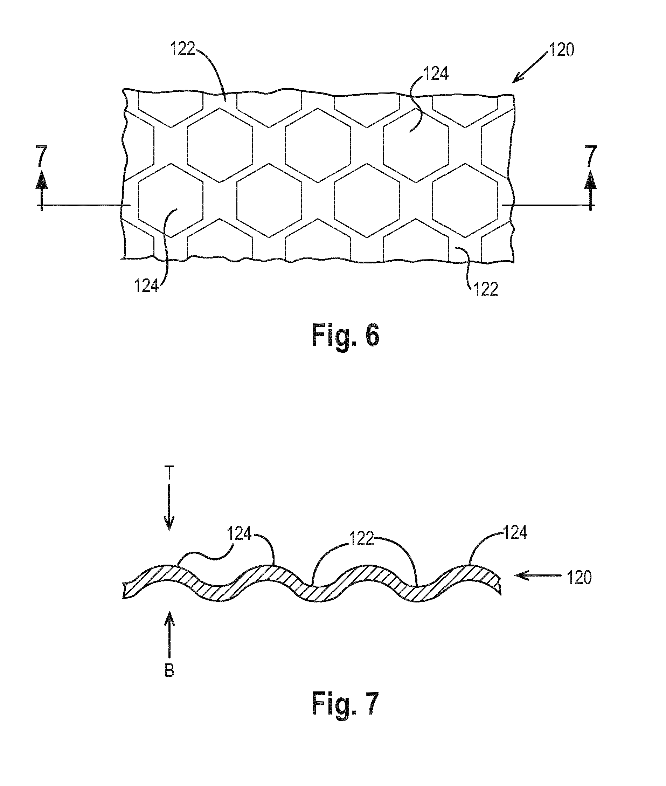

As exemplified in Figs. 6 and 7, a three-dimensional fibrous web 120, such as a wet-laid fibrous web, may be formed that has a plurality of protrusions extending outwardly from the plane of the fibrous web. The plane of the fibrous web may form a continuous or substantially continuous network region 122 while the plurality of protrusions form discrete zones 124 dispersed throughout the substantially continuous network region 122. The continuous network region may not be completely planar but may comprise indentations (indented into the page, i.e. towards the other side of the fibrous web relative to the plurality of discrete zones 124).

Alternatively to being continuous or substantially continuous, the network region may be substantially semi-continuous.

Fig. 7 is a cross-sectional view of the fibrous web 120 taken along line 7-7 of Fig. 6. As can be seen from the example of Fig. 7, the continuous network region 122 is essentially monoplanar. The plurality of discrete zones 124 are dispersed throughout the entire continuous network region 122 and essentially each discrete zone 124 is encircled by the continuous network region 122. The shape of the discrete zones 124 may be defined by the continuous network region 122. As shown in Fig. 7, the discrete zones 124, extend from (protrude from) the plane formed by continuous network region 122 toward an imaginary observer looking in the direction of arrow T of Fig. 7. When viewed by an imaginary observer looking in the direction indicated by arrow B of Fig. 7, the plurality of discrete zones 124 may comprise arcuately shaped voids which appear to be cavities or dimples.

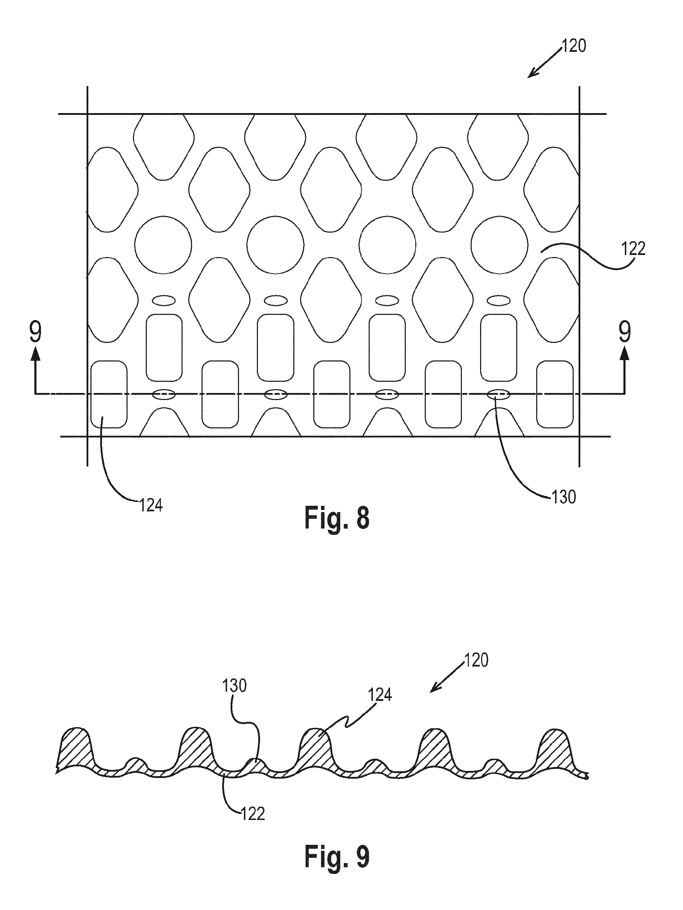

The plurality of protrusions may not all have the same height (i.e. the same caliper). This is exemplary shown in Figs. 8 and 9, where a first plurality of discrete zones 124 forms a first plurality of protrusions having a first caliper, and second plurality of discrete zones 130 forms a second plurality of protrusion having a second caliper. In the fibrous web shown in Figs. 8 and 9, the second caliper is smaller than the first caliper. In addition, the fibrous web may have third, fourth, fifth and further discrete zones forming further plurality of protrusion which differ from each other (and differ from the first and second caliper).

In one instance, three-dimensional fibrous web may be creped or uncreped. The continuous network region may have a first basis weight and the plurality of discrete zones may have a second, different basis weight. The continuous network region may have a first caliper or elevation and the plurality of discrete zones (i.e. the protrusions) may have a second caliper or elevation. The first and second calipers or elevations maybe different.

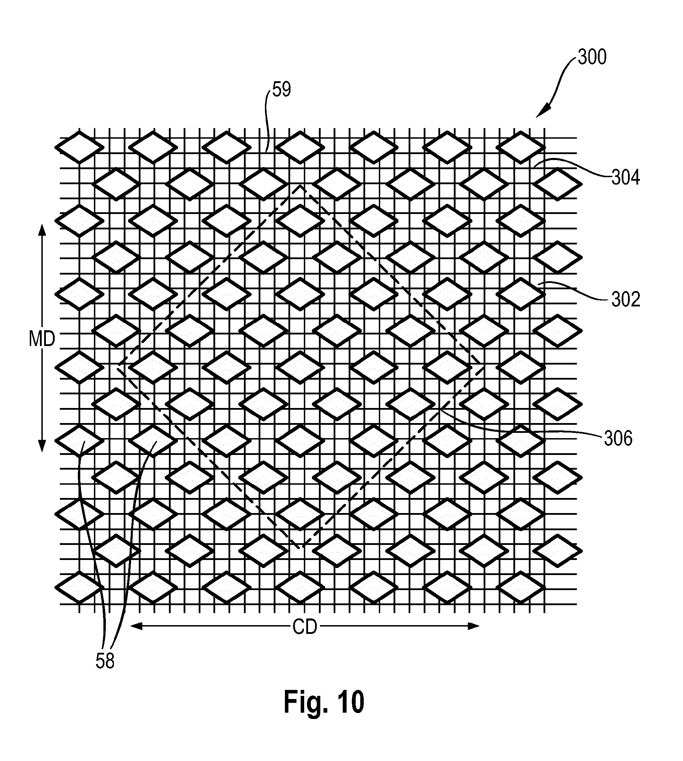

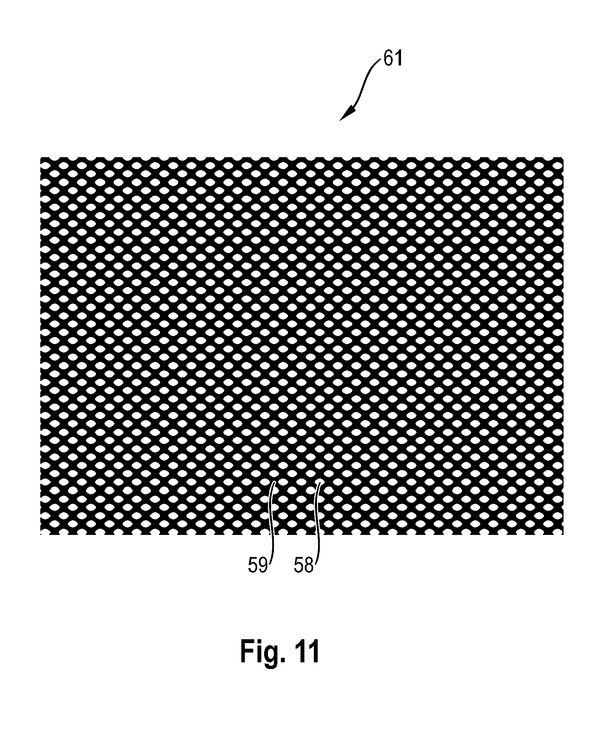

Referring to Fig. 10, a web of the fibrous web of the distribution system 54 maybe made through the use of a patterned papermaking belt 200 for forming three-dimensionally structured wet-laid and wet-formed webs as described in U.S. Patent No. 4,637,859, issued Jan. 20, 1987, to Trokhan. The papermaking belt 200 may comprise a reinforcing element 202 (such as a woven belt) which may be coated with an uncured liquid photosensitive polymeric resin to a preselected thickness. A film (as illustrated in example form in Fig. 11) incorporating a desired resin pattern may be juxtaposed on the liquid photosensitive resin. Note that the film of Fig. 11 would form ovate raised resin portions on the reinforcing element 202. The resin may then be exposed to light of an appropriate wave length, such as an ultraviolet wave length, through the film. This exposure to light causes curing of the resin in the exposed areas (i.e., white portions, clear portions, or non-printed portions in the film). Unexposed (and uncured) resin (under the black portions or printed portions of the film) is removed from the belt 200 by fluid flushing leaving behind the cured resin (e.g., elements 58 of Fig. 10) forming the desired pattern, which pattern then is used to form, during the wet-forming phase of papermaking, a fibrous web of the present disclosure. Because the pattern is transferred to the fibrous web while the web is still at least partially wet and then the web is subsequently dried (to set the pattern), the three-dimensional elements (e.g., continuous network region and plurality of discrete zones) formed in the fibrous web are locked by the setting associated with drying. Furthermore, the web comprises one or more wet strength resins, such as Kymene®, which functions to help lock the three-dimensional surface topography into the web via cross-linking. Thus, the three-dimensional elements of the web withstand losing their structure upon subsequent wetting by liquid bodily exudates and wearer induced strains and/or after receiving more than one insult of liquid bodily exudates.

The fibrous web 120 may be formed using the patterned papermaking belt 200 having the plurality of raised resin portions 58, each raised resin portion 58 forming a corresponding discrete element 124 (i.e. protrusion) in the fibrous web 120. The areas of the papermaking belt 200 that do not have the raised resin portions 58 form the continuous network region (i.e. the plane of the fibrous web) in the fibrous web 120.

Referring to Figs. 10 and 11, each raised resin portion 58 of the papermaking belt 200 may be surrounded by a substantially continuous or continuous deflection conduit 59, created when uncured resin is removed from the papermaking belt 200. The deflection conduit 59 is formed under black, printed and/or non-light penetrating portions of the film applied to the resin before resin curing. An example patterned film 61 used for curing select portions of the resin on the papermaking belt 200 is illustrated in Fig. 11. Portions of the patterned film 61 that are black, printed, and/or non-light penetrating represent portions of the patterned papermaking belt 200 that are resin free or substantially resin free after resin curing, while portions of the patterned film 61 that are white, clear, non-printed, and/or light penetrating represent portions of the patterned papermaking belt 200 that have cured resin forming the raised resin elements 58.

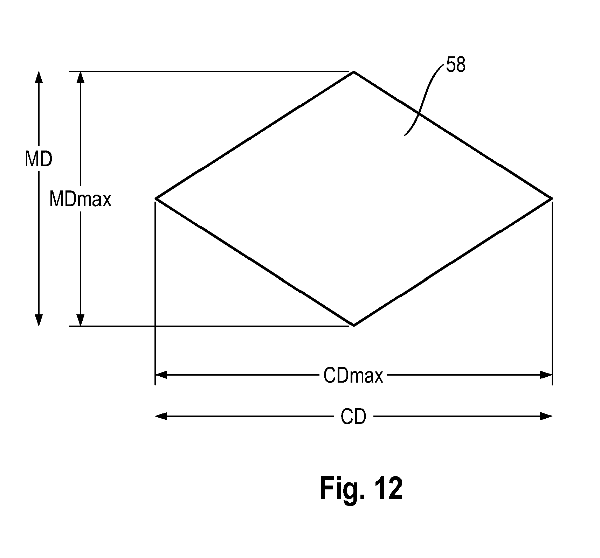

Referring again to Fig. 10, one unit 206 (shown by dashed line) of one example of a pattern of the papermaking belt 200 is illustrated. Referring to Fig. 12, a top view of an individual raised resin portion 58 that forms an individual discrete element. The raised resin portion 58 may have any suitable shape, such as a generally elongated shape having a major axis, $CD_{max}$, and a minor axis, $MD_{max}$. The raised resin portion 58 may also have any other suitable shape, such as round, ovate, square, rectangular, trapezoidal, or any other polygonal shape. As shown in the example of Fig. 12, individual raised resin portions 58 may have a rhomboid shape. One papermaking belt 200 may have more than one shape of raised resin portions. In general, the dimensions of the discrete elements 57 (i.e. protrusions) of the fibrous webs 120 are determined by the dimensions of the corresponding raised portions 58 that they are formed on. That is, the fibrous web is generally formed over the three-dimensional structure of the papermaking belt 200, so that in one sense the fibers are formed over, or molded to, the raised resin portions 58. If the raised resin portions form a continuous network, then the continuous network in the fibrous web may be formed on the raised resin portions, while the discrete elements will be formed in deflection conduits intermediate portions of the raised resin portions. Although referred to herein, as "raised resin portions", it is also within the scope of the present disclosure to use materials other than resin to form the raised portions on the papermaking belt in various processes with or without the film 61. Those processes are also within the scope of the present disclosure, if they are able to form the fibrous webs discussed herein.

In the raised resin portion 58 of Fig. 12, the ratio of the length of axis, $CD_{max}$, to the length of axis, $MD_{max}$, may be greater than or equal to one or less than 1. Stated another way, the axis, $CD_{max}$, may be longer than, shorter than, or may have the same length as the axis, $MD_{max}$. In one form, the ratio of the length of the axis, $CD_{max}$, to the length of the axis, $MD_{max}$, may be in the range of 1 to about 3 or in the range of 1 to about 4 or more.

In one form, the $CD_{max}$ of one raised resin portion 58 maybe between about 1.50 mm to about 3.50 mm, about 1.55 mm to about 2.00 mm, or about 1.53 mm and about 2.29 mm, and the $MD_{max}$ of one raised portion 58 may be between about 0.80 mm to about 2.00 mm, about 1.00 mm to about 1.70 mm, or about 1.01 mm to about 1.53 mm, specifically reciting al 0.01mm increments within the above-specified ranges and all ranges formed therein or thereby.

Any other suitable dimensions of the raised portions or raised resin portions are within the scope of the present disclosure, depending on the pattern desired for the fibrous web.

Some example shapes of the discrete zones (formed by the raised portions or raised resin portions) may comprise circles, ovals, squares, rectangles, ellipses, and polygons having any suitable number of sides. There is no requirement that the discrete zones be regular polygons or that the sides of the discrete zones 124 be straight. Instead, the discrete zones may comprise curved sides, stepped sides, or other multi-level sides.

Process of Making a Continuous Web of the Three-dimensional Fibrous Webs

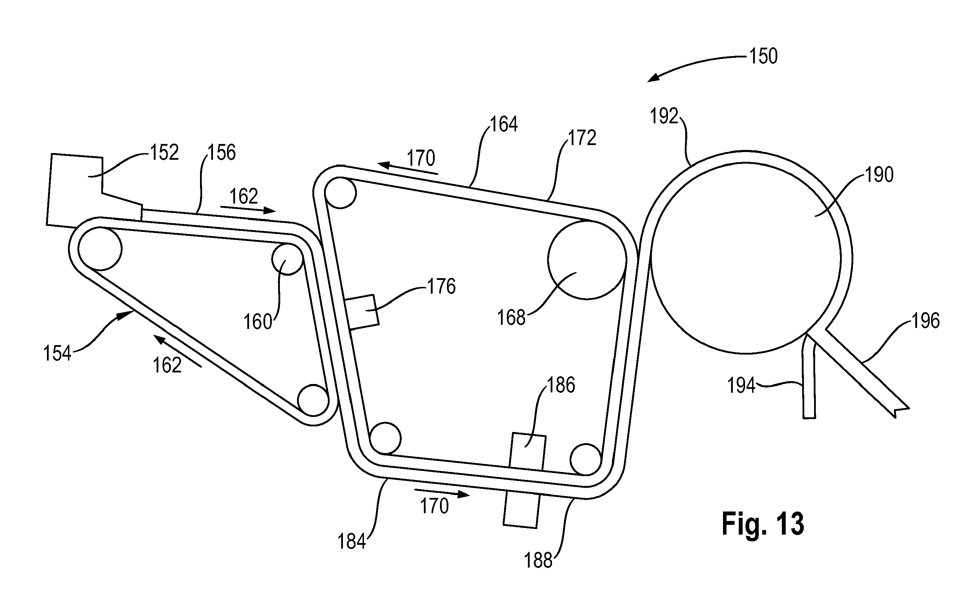

Fig. 13 is a simplified, schematic representation of one example of a continuous fibrous web making process and machine.

The example process, represented as 150, for making a continuous fibrous web of the distribution system comprises supplying an aqueous dispersion of fibers to a headbox 152. From the headbox 152, the aqueous dispersion of fibers may be delivered to a foraminous member 154 to produce an embryonic fibrous web 156. The foraminous member 154 may be conveyed in the direction indicated by arrow 162 by any suitable drive mechanism, such as a motor, for example (not illustrated).

After the aqueous dispersion of fibers is deposited onto the foraminous member 154, the embryonic fibrous web 156 is formed, typically by the removal of a portion of the aqueous dispersing medium by techniques known to those skilled in the art. The embryonic fibrous web 156 may then travel with the foraminous member 154 about return roll 160 and may be brought into contact with a papermaking belt 164. While in contact with the papermaking belt 164, the embryonic fibrous web 156 (and fibers thereof) may be deflected, rearranged, and/or further dewatered. The papermaking belt 164 maybe similar to the papermaking belt 200 discussed herein.

The papermaking belt 164 may be in the form of an endless belt that can travel in the direction indicated by directional arrow 170. The papermaking belt 164 may be constructed in such a manner that when water is caused to be removed from the embryonic fibrous web 156, as by the application of differential fluid pressure, such as by a vacuum box 176, and when the water is removed from the embryonic fibrous web 156 in the direction of the papermaking belt 164, the water can be discharged from the system without having to again contact the embryonic fibrous web 156 in either the liquid or the vapor state. A first surface 172 of the papermaking belt 164 may comprise one or more raised resin portions 58, as described herein. The raised portions may be discrete elements or form network regions.

After the embryonic fibrous web 156 has been associated with the papermaking belt 164, wet-laid fibers within the embryonic fibrous web 156 are deflected into the deflection conduits (or other non-resin or raised material containing areas on the papermaking belt, such as discrete zones) present in the papermaking belt member 164 to lead to an intermediate fibrous web 184.

The intermediate fibrous web 184 may first pass through an optional predryer 186. The predryer 186 maybe a conventional flow-through dryer (hot air dryer) known to those of skill in the art. The predried fibrous web 188 may travel to an impression nip roll 168. As the predried fibrous web 188 passes through the nip formed between impression nip roll 168 and a surface of a Yankee dryer 190, the pattern formed by the top surface 172 of the papermaking belt 164 is impressed into the predried fibrous web 188 to form a pattern of raised areas in the fibrous web 192. The raised areas (whether network regions or discrete zones) are formed in areas of the imprinted fibrous web 192 where little or no resin was present on the papermaking belt 164. The raised areas are low density areas of the imprinted fibrous web 192. High density areas (whether network regions or discrete zones) are formed in the imprinted fibrous web 192 where the resin or other material was present on the papermaking belt 164.

The imprinted fibrous web 192 may then be foreshortened by creping the web 192 with a creping blade 194 (or doctor blade) to remove the web 192 from the surface of the Yankee dryer 190 resulting in the production of a creped fibrous web 196. The continuous fibrous web 196 may be wound in a roll for later use in the manufacture of a distribution material or fed directly into a process for making the distribution material.

In other instances, the fibrous web 120 may be uncreped. Some techniques used to produce uncreped fibrous webs are taught in, for example, European Patent Application 0 677 612 A2, published on October 18, 1995, to Wendt, et al., European Patent Application 0 617 164 A1, published on September 28, 1994, to Hyland et al., and U.S. Patent No. 5,656,132, issued on August 12, 1997, to Farrington et al.

The continuous fibrous web may either be used for formation of the distribution system immediately after it has been made, or it can be wound up on a roll for intermediate storage and unwound later for further processing. The continuous fibrous web 196 may be cut into a plurality of appropriate sheets of fibrous webs 120. The continuous fibrous web may also be assembled with the layer of non-consolidated fibers (by providing the non-consolidated fibers on a central portion of the continuous fibrous web) and then the continuous distribution material may be cut into a plurality of appropriately sized sheets. First and second longitudinal portions (i.e. the portions of the fibrous web onto which no non-consolidated fibers have been deposited) may either be folded over onto the layer of non-consolidated fibers before or after the continuous distribution material is cut into a plurality of appropriately sized sheets. Upon folding of the longitudinal portions of the fibrous web (before or after cutting into individual sheets from the continuous fibrous web) onto the layer of non-consolidated fibers, the distribution system of the present invention is provided.

Layer of Non-Consolidated Fibers

The layer of non-consolidated fibers is deposited on a part or on the complete central portion of the fibrous web. The non-consolidated fibers maybe any fibers able to acquire and distribute fluid and release it into the absorbent core. The fibers may be synthetic, natural fibers, or combinations thereof.

The fibers of the layer of non-consolidated fibers may comprise or may consist of cellulose fibers, modified cellulose fibers, or combinations thereof. Modified cellulose fibers may be chemically modified cellulose fibers, such as intra-fiber cross-linked cellulose fibers. Such intra-fiber cross-linked cellulose fibers may have desirable absorbency properties. The layer of non-consolidated fibers may for example comprise at least 50%, or 60%, or 70%, or 80%, or 90% by weight of modified cellulose fibers, such as intra-fiber cross-linked cellulose fibers. Exemplary chemically intra-fiber cross-linked cellulose fibers are disclosed in US Patent No. 5,137,537. In certain embodiments, the chemically intra-fiber cross-linked cellulose fibers are intra-fiber cross-linked with between 0.5 mole % and 10.0 mole % of a $C_2$ to $C_9$ polycarboxylic cross-linking agent or between 1.5 mole % and about 6.0 mole % of a $C_2$ to $C_9$ polycarboxylic cross-linking agent based on glucose unit. Citric acid and polyacrylic acid are exemplary cross-linking agents. Further, according to certain embodiments, the intra-fiber cross-linked cellulose fibers have a water retention value of about 25 to 60, or 28 to 50, or 30 to 45. A method for determining water retention value is disclosed in US Patent No. 5,137,537. According to certain embodiments, the intra-fiber cross-linked cellulose fibers may be crimped, twisted, curled, or a combination thereof.

The intra-fiber cross-linked cellulose fibers may be provided by air-laying them on the central portion or a part of the central portion of the fibrous web.

The intra-fiber cross-linked cellulosic fibers provide high resilience and therefore relatively high resistance against the compression in the product packaging or in use conditions, e.g. under baby weight. This provides a relatively high void volume, permeability and liquid absorption, and hence helps to reduce leakage and improve dryness.

The layer of non-consolidated fibers may comprise the intra-fiber cross-linked cellulose fibers mixed with other fibers such as natural or synthetic polymeric fibers. According to exemplary embodiments, such other natural or synthetic polymeric fibers may include high surface area fibers, polyethylene fibers, polypropylene fibers, PET fibers, rayon fibers, lyocell fibers, and mixtures thereof.

Layers of non-consolidated fibers comprising a mixture of intra-fiber cross-linked cellulose fibers with other fibers may comprise 60 to 90% by weight of intra-fiber cross-linked cellulose fibers, 5 to 20 % by weight polyester (PET) fibers, and 5 to 20% by weight non-modified cellulose fibers. In another example, the layer of non-consolidated fibers may comprise 60 to 90% by weight intra-fiber cross-linked cellulose fibers, 5 to 20% by weight lyocell fibers, and 5 to 20% by weight PET fibers.

If the layer of non-consolidated fibers comprises different fibers, the different fibers (such as cellulose fibers, modified cellulose fibers, synthetic fibers) may be mixed with each other, they may be laid down one on top of the other in sub-layers or some types of fibers may be mixed and laid down with additional types of fibers or mixtures of different fibers being laid down on top of this sub-layer.

**Construction of the Distribution System**

The distribution system of the present invention is provided between the absorbent core and the topsheet of an absorbent article. The distribution system comprises a fibrous web having a central portion and first and second longitudinal portions. The central portion and first and second longitudinal portions are substantially parallel with the longitudinal dimension of the absorbent article.

The distribution system of the present invention may comprise more than one fibrous web. The distribution system may comprise from 1 to 10 fibrous webs, or from 1 to 5 fibrous webs, or from 2 to 5 fibrous webs. For example, the distribution system may comprise more than one fibrous web which are piled up one upon the other. One or more of the fibrous webs may be provided above the folded over longitudinal portions of the fibrous web which is folded over the layer of non-consolidated fibers, towards the topsheet of the absorbent article and/or one or more of the fibrous webs maybe provided below the central portion of fibrous web which is folded over the layer of non-consolidated fibers, towards the absorbent core of the absorbent article. Additionally or alternatively, one or more fibrous webs may be placed on top of each other and jointly folded over the layer of non-consolidated fibers with their respective first and second longitudinal portions.

Referring again to Fig. 1, one or more of the front region 541, back region 542, and/or the middle region 543 of the distribution system 54 may comprise one or more layers of the fibrous web, two or more layers of the fibrous web, three or more layers of the fibrous web, from 1 to 10 layers of the fibrous web, from 1 to 5 layers of the fibrous web, or from 2 to 5 layers of the fibrous web. One or more of the regions 541, 542, and 543 may have the same number of layers of the fibrous web or a different number of layers of the fibrous web. In one instance, one or more of the regions may have more or less layers than the remaining regions.

The distribution system further comprises a layer of non-consolidated fibers deposited on at least a part of the central portion of the fibrous web. The layer of non-consolidated fibers may also be provided on the complete central portion of the fibrous web. If the layer of non-consolidated fibers is only provided on a part of the central portion, the layer of non-consolidated fibers may have a smaller lateral /and or longitudinal extension compared to the central portion of the fibrous web. Alternatively or in addition, the layer of non-consolidated fibers may be deposited with interruptions, such that areas are formed on the central portion of the fibrous web, where no non-consolidated fibers are deposited and which areas are partly or fully surrounded by non-consolidated fibers.

Moreover, the basis weight of the layer of non-consolidated fibers may be homogeneous (disregarding possible areas where no non-consolidated fibers are deposited for the calculation of the basis weight). Alternatively, the basis weight of the layer of non-consolidated fibers may vary, for example the basis weight in the area coinciding with the crotch region of the absorbent article may have a higher basis weight than areas coinciding with the front and/or back region of the article.

The layer of non-consolidated fibers has a first surface and a second surface.

The first and second longitudinal portions of the fibrous web are folded over the layer of non-consolidated fibers such that the first surface of the layer of non-consolidated fibers is facing towards the central portion of the fibrous web (i.e. the first surface will typically be in contact with the central portion of the fibrous web) and the second surface of the layer of non-consolidated fibers is facing towards the first and second longitudinal portions of the fibrous web.

The fibrous web may partly enwrap the layer of non-consolidated fibers. If the fibrous web only partly enwraps the layer of non-consolidated fibers, the dimension of the first and second longitudinal portions is such that their combined lateral dimension is smaller than the lateral dimension of the central portion. Thereby, a gap is formed between the first and second longitudinal portions of the fibrous web on the second surface of the layer of non-consolidated fibers.

Alternatively, the fibrous web may fully enwrap the layer of non-consolidated fibers. If the fibrous web fully enwraps the layer of non-consolidated fibers, the dimension of the first and second longitudinal portions is such that their combined lateral dimension is at least as wide, typically wider than the lateral dimension of the central portion. Thereby, no gap is formed between the first and second longitudinal portions of the fibrous web on the second surface of the layer of non-consolidated fibers. If the combined lateral dimension of the first and second longitudinal portions is wider than the lateral dimension of the central portion, the first and second longitudinal portions will overlap when being folded over the layer of non-consolidated fibers. The first and second longitudinal portions may or may not be attached to each other in the area of overlap. If they are attached to each other, the attachment may be continuous or intermittent. Attachment can be facilitated by any means known in the art, such with adhesive or by ultrasonic bonding.

Fully enwrapping of the layer of non-consolidated fibers further helps to prevent escaping of fibers from the layer of non-consolidated fibers. This does not only apply to the manufacturing of the absorbent article but also to the absorbent article in use, especially if the absorbent article comprises an apertured topsheet. As fibers may be pulled out of the layer of non-consolidated fibers, the fibers may get onto the surface of the absorbent article and come into contact with the skin of the wearer. Such occurrences can be reduced (by partial enwrapping) or avoided (by full enwrapping) with the distribution system of the present invention.

If the distribution system comprises more than one fibrous web, only one, more than one or all of these fibrous webs may comprise a central portion and a first and second longitudinal portion. Alternatively, one or more than one of the fibrous webs may only have a central portion or may have a central portion and a first longitudinal portion, as long as at least one of the fibrous webs has a central portion and a first and second longitudinal portion in accordance with claim 1. Moreover, if the distribution system comprises more than one fibrous web, one or more of the fibrous webs may not be folded over the layer of non-consolidated fibers at long as at least one of the fibrous webs has its longitudinal portions folded in accordance with claim 1.

The first and second longitudinal portions of the fibrous web may each have a lateral dimension (i.e. width) of at least 10%, or at least 15%, or at least 20%, or at least 25%, or at least 30%, or at least 40%, or at least 50%, or at least 55%, or at least 60% of the lateral dimension of the central portion of the fibrous web. The lateral dimension of the first and second longitudinal portions of the fibrous web may each represent at least 10%, or at least 15%, or at least 20%, or at least 25%, or at least 30% of the total width of the fibrous web.

The fibrous web, the central portion and the first and second longitudinal portions may be rectangular. If any of the fibrous web, the central portion and/or the first and second longitudinal portions are not rectangular, the percentages given in the previous paragraph relate to the smallest lateral dimension of the fibrous web, the central portion, and the first and second portions, respectively.

The fibrous web may be wet laid, such as wet laid by the process described above.

The fibrous web may comprise cellulose fibers, modified cellulose fibers or combinations thereof. The fibrous web may comprise at least 80%, or at least 85%, or at least 90% by weight of the fibrous web of cellulose fibers, modified cellulose fibers or combinations of cellulose fibers and modified cellulose fibers. The fibrous web may comprise binders or additives. The fibrous web maybe free of synthetic fibers.

The fibrous web may have a three-dimensional surface topography, e.g. having a plurality of protrusions extending outwardly from the plane of the fibrous web. Using a fibrous web with a three-dimensional surface topography helps to immobilize the fibers of the layer of non-consolidated fibers. At least some of the fibers can be "trapped" in the three-dimensional topography of the fibrous web surface.

The three-dimensional surface topography of the fibrous web maybe facilitated as set out in detail above. The plane of the fibrous web may form a continuous or semi-continuous network region wherein a plurality of discrete zones forms the plurality of protrusions, which are dispersed throughout the continuous network region. The continuous or semi-continuous network region may have a first caliper and the plurality of discrete zones forming the protrusions may have a second caliper which is different from the first caliper. Generally, the second caliper may be larger than the first caliper. If the continuous network region does not have a homogeneous caliper, the average caliper of the continuous or semi-continuous network region will be the first caliper. The second caliper may be at least 40%, or at least 50%, or at least 80%, or at least 100%, or at least 120% higher than the first caliper (to be determined using samples wherein the cross-section is inspected using scanning electron microscopy).

If the distribution system comprises more than one fibrous web, the protrusions of the different fibrous webs may all face towards the same direction (i.e. towards the topsheet or towards the backsheet), or, alternatively, the protrusions of one or more fibrous web may face towards the topsheet while the protrusions of one or more other fibrous webs may face towards the backsheet. The plurality of protrusions of the fibrous web being in direct contact with the layer of non-consolidated fibers may face towards the layer of non-consolidated fibers or, alternatively, the plurality of protrusions may face away from the layer of non-consolidated fibers.

In still another alternative, the fibrous web may have protrusions extending outwardly of the plane of the fibrous web towards one direction (such as towards the topsheet) and may have further protrusions extending outwardly of the plane of the fibrous web towards the other direction (such as towards the backsheet). If the distribution system of the present invention comprises more than one fibrous web, this may apply to only one, more than one, or all of the fibrous webs.

The distribution system may further comprise adhesive, such as a hot melt adhesive, applied between the fibrous web and the layer of non-consolidated fibers. The adhesive will typically be applied onto the fibrous web prior to depositing the layer of non-consolidated fibers onto the central portion of the fibrous web. Application of adhesive can further assist in immobilizing the fibers of the layer of non-consolidated fibers.

The layer of adhesive may be applied discontinuously to avoid an adverse impact on the fluid handling properties (such as liquid permeability) of the distribution system. For example, less than 60%, or less than 50%, or less than 40%, or less than 30%, or less than 20% of the surface of the fibrous web facing towards the layer of non-consolidated fibers may be covered with adhesive. The area of the surface of the fibrous web facing towards the layer of non-consolidated fibers and covered with adhesive may not be less than 5%, or less than 10%, or less than 15%, or less than 20%, or less than 25% based on the total surface area of the fibrous web to facilitate appropriate immobilization of the fibers of the layer of non-consolidated fibers. If the fibrous web has a three-dimensional surface topography, the total surface area is the projected surface area as seen from above the fibrous web and looking at the fibrous web. The projected surface area will generally be smaller than the actual surface area for a fibrous web having a three-dimensional surface topography. Discontinuous application of adhesive can be facilitated by applying the adhesive in form of stripes, spirals, dots or any other form.

If the fibrous web has a three-dimensional surface topography and at least some or all of the protrusions face towards the layer of non-consolidated fibers, the adhesive can be applied only on the outermost areas (i.e. on the tips) of those protrusions facing towards the layer of non-consolidated fibers. In such embodiments, rather than using a noncontact application for the adhesive (i.e. application without direct contact between the equipment which applies the adhesive and the fibrous web, such as nozzles or dies) the adhesive can be applied on the surface of the fibrous web which will subsequently be in direct contact with the layer of non-consolidated fibers, by a contact application, such as slot coating, whereby the equipment which applies the adhesive is in direct contact with the fibrous web. Due to the 3-dimensional structure of the fibrous web, it is possible to apply the adhesive only on the outermost areas of protrusions which will subsequently face towards the layer of non-consolidated fibers.

If the distributions system comprises more than one fibrous web, adhesive, such as hot melt adhesive, may also be applied between the different fibrous webs.

The layer of non-consolidated fibers may comprise at least 80%, or at least 90%, or 100% of cellulose fibers, modified cellulose fibers or combinations thereof, by weight of the layer of non-consolidated fibers. Especially suitable fibers are intra-fiber cross-linked cellulose fibers. Further details on suitable fibers for the layer of non-consolidated fibers are provided above.

The layer of non-consolidated fibers may have the same longitudinal dimension as the longitudinal dimension of the fibrous web. Hence, the layer of non-consolidated fibers may be provided on the central portion of the fibrous web across the complete longitudinal length of the fibrous web.

Alternatively, the layer of non-consolidated fibers may have a shorter longitudinal dimension compared to the longitudinal dimension of the fibrous web. Thus, the fibrous web may extend laterally outwardly beyond the layer of non-consolidated fibers on both, the front and back lateral edge of the distribution system. In still another alternative, the layer of non-consolidated fibers may have a shorter longitudinal dimension compared to the longitudinal dimension of the fibrous web such that the fibrous web extends laterally outwardly beyond the layer of non-consolidated fibers in the rear lateral edge of the distribution system (i.e. the edge which is positioned towards the rear waist region of the absorbent article) while the layer of non-consolidated fibers extends towards the front lateral edge of the fibrous web (i.e. the edge which is positioned towards the front waist region of the absorbent article).

Such embodiments, where the layer of non-consolidated fibers has a shorter longitudinal dimension compared to the longitudinal dimension of the fibrous web, maybe beneficial, as the fibers of the layer of non-consolidated fibers may be relatively expensive. For example, intra-fiber cross-linked cellulose fibers are typically more expensive versus a wet-laid fibrous web made of (or comprising high amounts of) cellulose fibers. Also, towards the front and especially towards the rear end of the distribution system (which is typically placed towards the front and rear waist region, respectively), typically lower amounts of liquids are introduced into the diaper. Hence, the requirements on liquid acquisition and distribution are less critical in those areas. Given that the layer of non-consolidated fibers contributes to the overall thickness of the absorbent article, it is hence possible to provide absorbent articles which are especially thin towards the rear waist region (and possibly also towards the front waist region), thus providing good fit and comfort, without compromising on the fluid handling properties of the absorbent article.

Moreover, if the longitudinal dimension of the layer of non-consolidated fibers is shorter than the longitudinal dimension of the fibrous web, this helps to reduce the "escape" of fibers which have been deposited on the fibrous web and which are at least partly enwrapped by the fibrous web, especially if a fibrous web with a three-dimensional surface topography is used, which assists the immobilization of the layer of non-consolidated fibers.

If the layer of non-consolidated fibers has a shorter longitudinal dimension compared to the fibrous web, such that the fibrous web extends laterally outwardly beyond the layer of non-consolidated fibers, the first and second longitudinal portions of the fibrous web may be attached to the central portion of the fibrous web in the areas extending laterally outward beyond the layer of non-consolidated fibers. The attachment can be continuously or intermittently across the lateral dimensions of the distribution system. This further aids in immobilizing and sealing the layer of non-consolidated fibers within the distribution system. Attachment can be done by any suitable means known in the art, such as by adhesive (e.g. hot melt adhesive), ultrasonic bonding, pressure and/or heat sealing, or combinations thereof.

It may be desirable that the fibrous web of the distribution system has a relatively high opacity. Compared to absorbent articles which have a layer of air-laid fibers as distribution system (such as intra-fiber cross-linked cellulose fibers), a fibrous web, such as a wet-laid fibrous web typically has a higher opacity. Also, if the basis weight of the layer of air-laid fibers of the prior art is relatively low, it is difficult to obtain a homogeneous opacity. Non-homogeneous opacity is sometimes perceived as an indication of lower quality by the consumer. By at least partly enwrapping a layer of non-consolidated fibers with a fibrous web, a more homogeneous impression can be obtained due to the opacity of the fibrous web, which typically is homogeneous throughout the fibrous web.

Moreover, if the absorbent article comprises an absorbent core with high amounts of superabsorbent polymer particles and very little or no cellulose fibers, very thin absorbent cores are enabled. This may lead to areas in the absorbent articles which are relatively transparent. For example the back waist region of the absorbent article may become relatively transparent as the absorbent core in this region often comprises relatively low amounts of absorbent material, such as superabsorbent polymer particles (and little to no cellulose fibers). Again, a higher degree of transparency of the absorbent article is not desirable for many consumers. Thus, using a fibrous web in the distribution system which has a relatively high opacity can help to increased overall opacity of the absorbent article, especially in the back waist region.

The opacity of the fibrous web of the distribution system may be from 25% to 80%. If the distribution system comprises only one fibrous web, the opacity of the fibrous web may be relatively high, such as from 45% to 80%, or from 60% to 80%. However, if the distribution system comprises more than one fibrous web, a sufficient degree of opacity is provided by more than one fibrous web and the opacity of a single fibrous web out of the more than one fibrous webs can be rather low. If the distribution system comprises more than one fibrous web, the opacity of all fibrous webs overlaying each other is the more important measure compared to the opacity of a single fibrous web. Therefore, if the distribution system comprises more than one fibrous web, the opacity of all fibrous webs together maybe from 45% to 80%, or from 60% to 80%. In such circumstances, the test method set out below for measuring the opacity is modified accordingly by determining the opacity of a stack of fibrous webs (having the number of "layers" of fibrous webs as is present in the distribution system) instead of measuring a single fibrous web only.

If the distribution system has regions with differing number of fibrous webs overlaying each other, the area having the highest number of fibrous webs overlaying each other is taken into account for the opacity. This shall take into account the central portion and the first and second longitudinal portions, which, as they overlay each other, shall be considered as two "layers" for distribution systems which have more than one fibrous web.

The basis weight of the distribution system maybe from 100 to 500 $g/m^2$, or from 200 to 300 $g/m^2$. The basis weight of the fibrous web (or combined basis weight of all fibrous webs if more than one fibrous web is used) may be from 10% to 90%, or from 20% to 80% by weight, or from 20% to 60% by weight of the total basis weight of the distribution system. The basis weight of the layer of non-consolidated fibers maybe from 10% to 90%, or from 20% to 80% by weight, or from 40% to 80% by weight of the total basis weight of the distribution system.

The basis weight of one fibrous web may be from 10 to 80 $g/m^2$, or from 15 $g/m^2$ to 60 $g/m^2$. The average basis weight of the layer of non-consolidated fibers comprised by the distribution system may be at least 60 $g/m^2$, or at least 70 $g/m^2$, or at least 80 $g/m^2$.

The average basis weight of the layer of non-consolidated fibers comprised by the distribution system may be less than 300 $g/m^2$, or less than 250 $g/m^2$, or less than 200 $g/m^2$, or less than 150 $g/m^2$. The basis weight of the layer of non-consolidated fibers may be homogeneous throughout the area where the non-consolidated fibers are provided. Alternatively, the basis weight of the layer of non-consolidated fibers may vary throughout the area were the non-consolidated fibers are provided. For example, the basis weight of the layer of non-consolidated fibers may be higher in the area positioned in the crotch region and/or the front waist region of the absorbent article compared to the basis weight in the area positioned in the back waist region.

The absorbent article may further comprise an acquisition layer positioned between the topsheet and the distribution system. The acquisition layer may have a shorter longitudinal dimension compared to the longitudinal dimension of the distribution system such that the distribution system extends laterally outwardly beyond the acquisition system towards the back waist region of the absorbent article. In such cases, a relatively high opacity of the fibrous web of the distribution system may be even more desirable. The distribution system may be provided in the absorbent article such that the central portion of the fibrous web faces towards the topsheet and the first and second longitudinal portions faces towards the absorbent core. Alternatively, the first and second longitudinal portions of the fibrous web may face towards the topsheet and the central portion may face towards the absorbent core.

The configuration wherein the central portion faces towards the topsheet may be more desirable compared the configuration wherein the first and second longitudinal portions face towards the topsheet, especially if the fibrous web is colored or is provided with a printed pattern or graphic. The fibrous web may provided with a printed pattern or graphic at least in the central portion on the surface of the fibrous web which is opposite to the surface on which the layer of non-consolidated fibers has been provided. Alternatively or in addition to providing a printed pattern or graphic, the fibrous web may be colored. This may be achieved by providing pigments, dyes or other materials known in the art in the fibrous web, e.g. by coloring the cellulose fibers, using colored binders or by otherwise adding pigments, dyes or the like. The central portion presents a more even, homogeneous surface compared to the surface of the distribution system with the first and second longitudinal portions. This applies especially if the first and second longitudinal portions do not cover the complete second surface of the layer of non-consolidated fibers, such that there is a gap between the first and second longitudinal portions where the layer of non-consolidated fibers is visible.

Having a distribution system comprising one or more visual features may provide one or more visual signals to the caregiver from the wearer-facing surface of the absorbent article that may help indicate the remaining absorbent capacity of the absorbent article, for example. The one or more visual features may also provide one or more visual signals that maybe configured to aid the consumer to in selecting an appropriate absorbent article for an appropriate time (e.g., daytime wear, nighttime wear).

In addition to or instead of providing the distribution system with one or more visual features, the distribution system, or portions thereof, may be embossed, by providing discrete embossed points that are typically circular or ovate. Embossing the distribution system may help improving the depth perception of the absorbent article when viewing the absorbent article from the wearer-facing surface thereof.

A distribution system solely made of unconsolidated air-laid fibers may form some cracks and disruptions especially when insulted with liquid bodily exudates and undergoes strain caused by the wearer's movement. These disruptions are mainly due to the fact that the unconsolidated air-laid fibers may not be able to provide enough wet integrity. As a result, undesired randomly positioned liquid channels in the distribution material 54 may occur and be observed as distribution material tears or separations. The liquid bodily exudates may be routed through these liquid channels directly to the absorbent core 28. As a consequence, the liquid bodily exudates may not be efficiently distributed in the distribution material if unconsolidated air-laid fibers are used. Moreover, these uncontrolled disruptions may be perceived as sign of lower product quality from some consumers.

In the case of the present disclosure, where a layer of non-consolidated fibers is partly or fully enwrapped by a fibrous web, such as a wet-laid fibrous web, the distribution system may have an improved cohesive structure or wet strength compared to a sole layer of unconsolidated air-laid fibers discussed above. Due to the wet-laid and wet-formed papermaking process as described herein, the wet-laid and wet-formed fibers relatively strongly adhere to each other. The fibrous web(s) of the distribution system of the present disclosure is/are thus less prone to form disruptions when wetted by liquid bodily exudates. Hence, the distribution system, of the present disclosure, comprising one or more layers of fibrous webs has an improved wet integrity relative to unconsolidated air-laid fibers. Liquid bodily exudates are therefore more efficiently distributed in the distribution material before being transferred to the absorbent core 28. Furthermore, due to the papermaking process as described herein, the wet-laid and wet-formed fibrous webs of the present disclosure better retain their three-dimensional surface topography, not only upon liquid bodily exudates wetting, but also upon wearer induced strains. This is the due to the fact that the fibrous webs are wet molded (i.e., wet-formed) and then dried to set the three-dimensional surface topography in addition to the provision of one or more wet strength resins.

A top portion of the distribution system 54 may be attached to the topsheet 24 or to any layer, e.g. the acquisition layer 52, which is positioned between the topsheet 24 and the distribution system 54. A bottom portion of the distribution system 54 maybe attached to a wearer-facing portion or web of the absorbent core 28 or to any layer between the distribution system 54 and the wearer-facing portion of the absorbent core 28. In other forms, the distribution system 54 may not be attached to any of the above-described layers and may merely be positioned there between without actual attachment (e.g., no gluing, bonding etc.).

The top portion of the distribution system 54 maybe attached to the topsheet 24 or to any layer between the topsheet 24 and the distribution system 54 by a patterned adhesive. The patterned adhesive may comprise one or more colors. The bottom portion of the distribution system 54 may be attached to a portion or web of the absorbent core 28 facing the topsheet 24 or to any layer between the portion or the web of the absorbent core 28 facing the topsheet 24 and the distribution system 54 by the same or a different patterned adhesive.

The patterned adhesive may comprise a plurality of adhesive lines that are continuous or discontinuous, linear or non-linear. The lines may extend in any suitable direction, such as longitudinally or laterally, for example.

In one instance, the patterned adhesive may be a plurality of overlapping, substantially continuous, semi-cycloidal lines of adhesive extending along the longitudinal direction, lateral direction, or other direction of the absorbent article 20.

In other instances, the patterned adhesive may be a plurality of separate lines, such as straight lines, spirals, and/or may be a plurality of dots of adhesive.

If the adhesive is hydrophobic (e.g., some hotmelt adhesives), the liquid bodily exudates may be drained from the distribution system 54 to the absorbent core 28 between the patterned adhesive.

The distribution system of the present invention may comprise less than 5% by weight of superabsorbent polymer particles based on the total weight of the distribution system, or may be free of superabsorbent polymer particles.

**Sanitary Napkin**

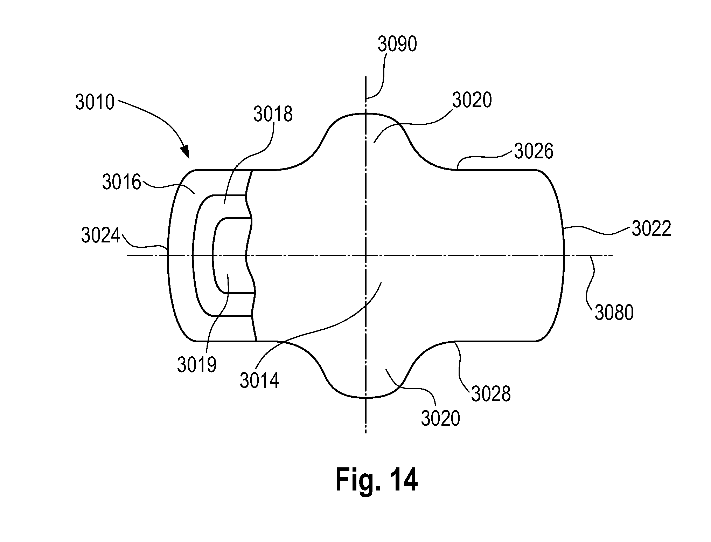

Referring to Fig. 14, the absorbent articles described herein may be a sanitary napkin 3010. The sanitary napkin 3010 may comprise a liquid permeable topsheet 3014, a liquid impermeable, or substantially liquid impermeable, backsheet 3016, and an absorbent core 3018. The absorbent core 3018 may have any or all of the features described herein. The sanitary napkin may comprise a secondary topsheet 3019. Either in addition to or in lieu of the second topsheet 3019, the sanitary napkin may comprise the distribution system of the present disclosure. The sanitary napkin 3010 comprises a lateral axis 3090 and a lateral dimension parallel to the lateral axis 3090. The sanitary napkin 3010 may also comprise wings 3020 extending outwardly with respect to a longitudinal axis 3080 of the sanitary napkin 3010. The wings 3020 maybe joined to the topsheet 3014, the backsheet 3016, and/or the absorbent core 3018. The sanitary napkin 3010 also comprises a front edge 3022, a rear edge 3024 longitudinally opposing the front edge 3022, a first side edge 3026, and a second side edge 3028 longitudinally opposing the first side edge 3026. The longitudinal axis 3080 extends from a midpoint of the front edge 3022 to a midpoint of the rear edge 3024 and the longitudinal dimension is parallel to the longitudinal axis 3080. The lateral axis 3090 extends from a midpoint of the first side edge 3028 to a midpoint of the second side edge 3028. The sanitary napkin 3010 may also be provided with additional features commonly found in sanitary napkins as is known in the art.

**Packages**

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

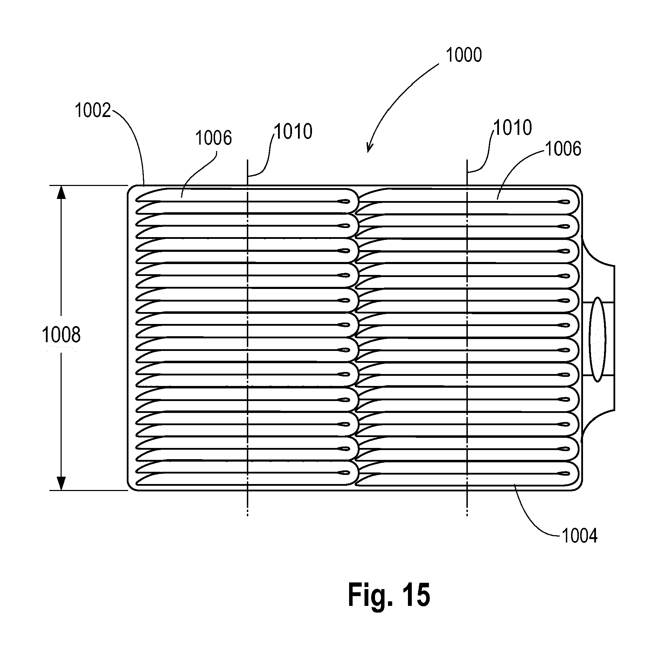

Fig. 15 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

**Test methods**

**Opacity test method**

Opacity is a measure of the capacity of a material to obscure the background behind it. The value for opacity is obtained by dividing the reflectance obtained with a black backing (RB) for the material, by the reflectance obtained for the same material with a white background (WB). This is called the contrast ratio (CR) method.

$$\% \text{ Opacity} = \frac{\text{RB}}{\text{RW}} \times 100$$

Using a Hunter Colorimeter set to XYZ color scale, opacity is defined as

$$\% \text{ Opacity} = \frac{\text{Y reading over black plate}}{\text{Y reading over white plate}} \times 100$$

Sample Preparation

A specimen of suitable size (generally about 10 cm square, but can be smaller if the sample is taken from an absorbent article and a continuous sample area of 10 cm square is not available) is cut for analysis. The specimen must be free of creases, wrinkles, tears and other obvious defects.

The test is conducted on samples that have been conditioned at a temperature of 23°C $\pm$ 1C° and a relative humidity of 50% ± 2% for a minimum of 2 hours prior to testing. Further, all tests are conducted in such conditioned room.

If the fibrous web of the distribution system does not have homogeneous opacity, a number of representative samples are taken from different locations in the fibrous web and their opacity is determined. The opacity of the fibrous web is then defined to be the average value of the opacity values obtained for the several representative samples.

Equipment

Hunter Labscan® XE available from Hunter Associates Laboratory, Inc., USA. The instrument is configured as follows:

Geometry 45°/0°
Color Scale XYZ
Illuminant D65
Observer 10°

The colorimeter is calibrated using the standard gloss black glass and gloss white tile supplied with the instrument according to the manufacturer's instructions.

Test Procedure

The specimen is placed on the white tile and inserted into the colorimeter according to the manufacturer's instructions. The machine direction of the specimen should be aligned front-to-back in the instrument. The Y reading is recorded to the nearest 0.1 unit. The procedure is repeated using the black standard plate instead of the white standard tile.

Ten specimens are measured and the opacity results averaged to obtain the % opacity value for the material.

$$\% \text{ Opacity} = \frac{\text{"Y" on black plate}}{\text{"Y" on white plate}} \times 100$$

**Wet Burst Strength Test Method**

The test is conducted on samples that have been conditioned at a temperature of 23°C ± 1C° and a relative humidity of 50% ± 2% for a minimum of 2 hours prior to testing. Further, all tests are conducted in such conditioned room.

The wet burst strength as used herein is a measure of the ability of the fibrous web of the distribution system to absorb energy, when wet and subjected to deformation with regard to the plane of the fibrous web.

The wet burst strength of a fibrous web (referred to as "sample" within this test method) is determined using an electronic burst tester and specified test conditions. The results obtained are averaged out of 4 replicates and the wet burst strength is reported for a fibrous web consisting of a single layer of wet-laid fibers.

Equipment

- Apparatus: Burst Tester - Thwing-Albert Vantage Burst Tester or equivalent ball burst instrument where the ball moves downward during testing. Refer to manufacturer's operation and set-up instructions. The ball diameter is 1.59 cm and the clamp opening diameter is 8.9 cm.

- Calibration Weights - Refer to manufacturer's calibration instructions

Laboratory testing:

- Paper Cutter - Cutting board, 600 mm size

- Scissors - 100 mm, or larger

- Pan - Approximate Width/Length/Depth: 240 x 300 x 50 mm, or equivalent

- Distilled water at the temperature of the conditioned room used

<u>Sample Preparation</u>

Cut the samples so that they are 228 mm in length and width of 140 mm in width.

<u>Operation</u>

Set-up and calibrate the Burst Tester instrument according to the manufacturer's instructions for the instrument being used.

Holding the sample by the narrow edges, the center of the sample is dipped into a pan filled approximately 25 mm from the top with distilled water. The sample is left in the water for 4 ($\pm$ 0.5) seconds.

The excess water is drained from the sample for 3 ($\pm$ 0.5) seconds holding the sample in a vertical position.

The test should proceed immediately after the drain step. The sample should have no perforations, tears or imperfections in the area of the sample to be tested. If it does, start the test over.

The sample is placed between the upper and lower rings of the Burst Tester instrument. The sample is positioned centered and flat on the lower ring of the sample holding device in a manner such that no slack in the sample is present.

The upper ring of the pneumatic holding device is lowered to secure the sample.

The test is started. The test is over at sample failure (rupture) i.e., when the load falls 20g from the peak force. The maximum force value is recorded.

The plunger will automatically reverse and return to its original starting position.

The upper ring is raised in order to remove and discard the tested sample.

The procedure is repeated until all 4 replicates have been tested.

<u>Calculation</u>

$$\text{Wet Burst Strength} = \text{sum of peak load readings / number of replicates tested}$$

Report the Wet Burst results to the nearest gram.

**In-Bag Stack Height Test**

The in-bag stack height of a package of absorbent articles is determined as follows:

The absorbent article package is conditioned at a temperature of 23°C $\pm$ 1C° and a relative humidity of 50% $\pm$ 2% for a minimum of 2 hours prior to testing.

<u>Equipment</u>

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ Igrams.

<u>Test Procedure</u>

Absorbent article packages are equilibrated at 23 $\pm$ 2 °C and 50 $\pm$ 5 % relative humidity prior to measurement. The horizontal sliding plate is raised and an absorbent article package is placed centrally under the horizontal sliding plate in such a way that the absorbent articles within the package are in a horizontal orientation (see Fig. 15). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any embodiment disclosed or claimed herein or that it alone, or in any combination with any other reference or references, teaches, suggests or discloses any such embodiment. Further, to the extent that any meaning or definition of a term in this document conflicts with any meaning or definition of the same term in a document incorporated by reference, the meaning or definition assigned to that term in this document shall govern.

While particular embodiments of the present disclosure have been illustrated and described, it would be obvious to those skilled in the art that various other changes and modifications may be made without departing from the spirit and scope of the present disclosure. It is therefore intended to cover in the appended claims all such changes and modifications that are within the scope of this disclosure.

## Claims

1. An absorbent article (20) having a longitudinal dimension and a lateral dimension and comprising:

   a. a liquid permeable topsheet (24);
   b. a liquid impermeable backsheet (25);
   c. an absorbent core (28) positioned between the topsheet (24) and the backsheet (25); and
   d. a distribution system (54) positioned between the absorbent core (28) and the topsheet (24), the distribution system (54) comprising

      i. a fibrous web (120) having a central portion (150) and first and second longitudinal portions (151, 152), the central portion (150) and first and second longitudinal portions (151, 152) being substantially parallel with the longitudinal dimension of the absorbent article (20), and
      ii. a layer of non-consolidated fibers (121) deposited on at least a part of the central portion (150) of the fibrous web (120), the layer having a first and a second surface,
      wherein the first and second longitudinal portions (151, 152) of the fibrous web (120) are folded over the layer of non-consolidated fibers (121) along the longitudinal dimension of the absorbent article (20), such that the first and second longitudinal portions (151, 152) are facing towards the second surface of the layer of non-consolidated fibers (121) and the central portion (150) is facing towards the first surface of the layer of non-consolidated fibers (121).

2. The absorbent article (20) of claim 1, wherein the fibrous web (120) comprises at least 80% cellulose fibers, modified cellulose fibers, or combinations of cellulose fibers and modified cellulose fibers, by weight of the fibrous web.

3. The absorbent article (20) of any of the preceding claims, wherein the fibrous web (120) is wet laid.

4. The absorbent article (20) of any of the preceding claims, wherein the fibrous web (120) has a three-dimensional surface topography.

5. The absorbent article (20) of claim 4, wherein the three-dimensional fibrous web (120) comprises a continuous network region (122) forming the plane of the fibrous web, and a plurality of discrete zones (124) forming a plurality of protrusions, wherein the discrete zones (124) are dispersed throughout the continuous network region (122).

6. The absorbent article (20) of claim 5, wherein the continuous network region (122) comprises a first average caliper;

and the plurality of discrete zones (124) comprises a second average caliper, and wherein the second average caliper is higher than the first average caliper.

7. The absorbent article (20) of any of claims 5 or 6, wherein at least the majority of the plurality of protrusions (124) faces towards the layer of non-consolidated fibers (121), and wherein the distribution system (54) further comprises adhesive between the fibrous web (120) and the layer of non-consolidated fibers (121), whereby the adhesive is applied only onto protrusions (124) of the fibrous web (124).

8. The absorbent article (20) of any of the preceding claims, wherein the non-consolidated fibers (121) comprise at least 80% cellulose fibers, modified cellulose fibers, or a combination of cellulose fibers and modified cellulose fibers by weight of the non-consolidated fibers.

9. The absorbent article (20) of any of the preceding claims, wherein the fibrous web (120) is folded over the layer of non-consolidated fibers (121) such that the first and second longitudinal portions (151, 152) partly overlap each other to fully enwrap the layer of non-consolidated fibers (121).

10. The absorbent article (20) of any of the preceding claims, wherein the layer of non-consolidated fibers (121) has a shorter lateral dimension compared to the fibrous web (120), such that the fibrous web extends laterally outwardly beyond the layer of non-consolidated fibers.

11. The absorbent article (20) of claim 10, wherein the first and second longitudinal portions (151, 152) of the fibrous web (120) are attached to the central portion (150) of the fibrous web (120) in at least a part of the areas extending laterally outward beyond the layer of non-consolidated fibers (121).

12. The absorbent article (20) of any of the preceding claims, wherein the absorbent article further comprises an acquisition layer (52) positioned between the topsheet (24) and the distribution system (54).

13. The absorbent article of any of the preceding claims, wherein the fibrous web (120) has an opacity from 25% to 80%.

14. The absorbent article of any of the preceding claims, wherein the central portion (150) of the fibrous web (120) faces towards the topsheet (24) and the first and second longitudinal portions (151, 152) of the fibrous web (120) face toward the absorbent core (28).

15. A package comprising a plurality of the absorbent articles (20) of any of the preceding claims, wherein the package has an in-bag stack height of less than about 110 mm, according to the In-Bag Stack Height Test herein.

# Fig. 1

Fig. 2

# Fig. 3

**Fig. 4**

EP 3 178 458 A1

**Fig. 5**

Fig. 6

Fig. 7

120

122

9          9

124          130

**Fig. 8**

130          124          120

122

**Fig. 9**

Fig. 10

61

59 58

**Fig. 11**

**Fig. 12**

Fig. 13

Fig. 14

**Fig. 15**

Europäisches
Patentamt

European
Patent Office

Office européen
des brevets

## EUROPEAN SEARCH REPORT

Application Number

EP 15 19 8435

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2016/040090 A1 (PROCTER & GAMBLE [US]) 17 March 2016 (2016-03-17) <br> * page 9, line 9 - line 16 * <br> * page 14, line 11 - page 15, line 6 * <br> * page 21, line 1 - page 23, line 27 * <br> * page 26, line 19 - page 27, line 23 * <br> * page 29, line 28 - page 31, line 22; figures 1,2,9-12B * <br> ----- | 1 | INV. <br> A61F13/537 |
| A | EP 2 740 454 A1 (PROCTER & GAMBLE [US]) 11 June 2014 (2014-06-11) <br> * paragraph [0021] * <br> * paragraph [0071] - paragraph [0081] * <br> * paragraph [0087] - paragraph [0092]; figures 1-3 * <br> ----- | 1-15 | |
| A | US 4 637 859 A (TROKHAN PAUL D [US]) 20 January 1987 (1987-01-20) <br> * column 2, line 20 - line 24 * <br> * column 18, line 33 - column 20, line 14; figures 6-9 * <br> ----- | 1-15 | |
| A,D | US 5 137 537 A (HERRON CARLISLE M [US] ET AL) 11 August 1992 (1992-08-11) <br> * the whole document * <br> ----- | 1-15 | TECHNICAL FIELDS SEARCHED (IPC) <br><br> A61F |

The present search report has been drawn up for all claims

| Place of search | Date of completion of the search | Examiner |
|---|---|---|
| Munich | 31 May 2016 | Demay, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 19 8435

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2016

| Patent document cited in search report | | Publication date | Patent family member(s) | | Publication date |
|---|---|---|---|---|---|
| WO 2016040090 | A1 | 17-03-2016 | NONE | | |
| EP 2740454 | A1 | 11-06-2014 | CA | 2894680 A1 | 19-06-2014 |
| | | | CN | 104853707 A | 19-08-2015 |
| | | | EP | 2740454 A1 | 11-06-2014 |
| | | | JP | 2016502871 A | 01-02-2016 |
| | | | US | 2014163504 A1 | 12-06-2014 |
| | | | WO | 2014093323 A1 | 19-06-2014 |
| US 4637859 | A | 20-01-1987 | NONE | | |
| US 5137537 | A | 11-08-1992 | NONE | | |

EPO FORM P0459

For more details about this annex : see Official Journal of the European Patent Office, No. 12/82

**EP 3 178 458 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3860003 A
- US 5221274 A
- US 5554145 A
- US 5569234 A
- US 5580411 A
- US 6004306 A
- US 5151092 A, Buell
- US 5599335 A, Goldman
- EP 1447066 A1, Busam
- WO 9511652 A, Tanzer
- US 5650214 A, Handoff
- WO 2012052172 A, Van Moldered
- US 7744576 B
- US 20110268932 A1
- US 20110319848 A1
- US 20110250413 A1
- EP 2740449 A1
- US 3700623 A
- US 3772076 A
- US 4557801 A
- US 4391878 A
- US 4637859 A, Trokhan
- EP 0677612 A2, Wendt
- EP 0617164 A1, Hyland
- US 5656132 A, Farrington
- US 5137537 A